# EUROPEAN PATENT SPECIFICATION

(11) **EP 2 397 152 B1**
(45) Date of publication and mention of the grant of the patent: **28.05.2014**
(21) Application number: 10710061.2
(22) Date of filing: 10.02.2010
(51) Int. Cl.: A61K 38/19

(54) **USE OF CARDIOTROPHIN- 1 FOR THE TREATMENT OF METABOLIC DISEASES**
VERWENDUNG VON CARDIOTROPHIN-1 ZUR BEHANDLUNG VON STOFFWECHSELERKRANKUNGEN
UTILISATION DE LA CARDIOTROPHINE-1 POUR LE TRAITEMENT DE MALADIES MÉTABOLIQUES

(30) Priority: 12.02.2009 ES 200900396
(43) Date of publication of application: 21.12.2011
(73) Proprietor: Proyecto de Biomedicina Cima, S.L., 31008 Pamplona Navarra (ES); Universidad de Navarra, 31009 Pamplona (Navarra) (ES)
(72) Inventor: PRIETO VALTUEÑA, Jesús María, E-31008 Pamplona - Navarra (ES); BUSTOS DE ABAJO, Matilde, E-31008 Pamplona - Navarra (ES); MORENO ALIAGA, María Jesús, E-31008 Pamplona - Navarra (ES)
(74) Representative: ABG Patentes, S.L.
(86) International application number: PCT/ES2010/070072
(87) International publication number: WO 2010/092218

(56) References cited:
- MARCOS-GÓMEZ B ET AL: "[Obesity, inflammation and insulin resistance: role of gp 130 receptor ligands]" May 2008 (2008-05), ANALES DEL SISTEMA SANITARIO DE NAVARRA 2008 MAY-AUG LNKD- PUBMED:18953360, VOL. 31, NR. 2, PAGE(S) 113 - 123 , XP002601360 ISSN: 1137-6627 * abstract
- ZVONIC SANJIN ET AL: "Effects of cardiotrophin on adipocytes." 12 November 2004 (2004-11-12), THE JOURNAL OF BIOLOGICAL CHEMISTRY 12 NOV 2004 LNKD- PUBMED:15339920, VOL. 279, NR. 46, PAGE(S) 47572 - 47579 , XP002601361 ISSN: 0021-9258 * abstract *Discussion*
- FEBBRAIO M A: "gp130 receptor ligands as potential therapeutic targets for obesity" JOURNAL OF CLINICAL INVESTIGATION, AMERICAN SOCIETY FOR CLINICAL INVESTIGATION, US LNKD- DOI:10.1172/JCI30453, vol. 117, no. 4, 2 April 2007 (2007-04-02) , pages 841-849, XP008121201 ISSN: 0021-9738
- WATT MATTHEW J ET AL: "CNTF reverses obesity-induced insulin resistance by activating skeletal muscle AMPK." May 2006 (2006-05), NATURE MEDICINE MAY 2006 LNKD- PUBMED:16604088, VOL. 12, NR. 5, PAGE(S) 541 - 548 , XP002601362 ISSN: 1078-8956 * abstract
- JUNG CHRISTIAN ET AL: "Cardiotrophin-1 in adolescents: impact of obesity and blood pressure." August 2008 (2008-08), HYPERTENSION AUG 2008 LNKD- PUBMED:18541732, VOL. 52, NR. 2, PAGE(S) E6; AUTHOR REPLY E7 , XP002601363 ISSN: 1524-4563 the whole document
- HONGKUI J ET AL: "In vivo effects of cardiotrophin-1", CYTOKINE, ACADEMIC PRESS LTD, PHILADELPHIA, PA, US, vol. 8, no. 12, 1 December 1996 (1996-12-01), pages 920-926, XP002966035, ISSN: 1043-4666, DOI: 10.1006/CYTO.1996.0123
- PATEL SANJEEV B ET AL: "Leptin: linking obesity, the metabolic syndrome, and cardiovascular disease.", April 2008 (2008-04), CURRENT HYPERTENSION REPORTS APR 2008 LNKD- PUBMED:18474180, VOL. 10, NR. 2, PAGE(S) 131 - 137 ISSN: 1534-3111
- WHITE URSULA A ET AL: "Neuropoietin attenuates adipogenesis and induces insulin resistance in adipocytes.", 15 August 2008 (2008-08-15), THE JOURNAL OF BIOLOGICAL CHEMISTRY 15 AUG 2008 LNKD- PUBMED:18562323, VOL. 283, NR. 33, PAGE(S) 22505 - 22512 ISSN: 0021-9258
- NATAL CRISTINA ET AL: "Cardiotrophin-1 is expressed in adipose tissue and upregulated in the metabolic syndrome.", January 2008 (2008-01), AMERICAN JOURNAL OF PHYSIOLOGY. ENDOCRINOLOGY AND METABOLISM JAN 2008 LNKD- PUBMED:17940213, VOL. 294, NR. 1, PAGE(S) E52 - E60 ISSN: 0193-1849

## Description

### TECHNICAL FIELD OF THE INVENTION

The invention is related to the use of cardiotrophin-1 (CT-1) for the treatment of obesity and associated disorders: hyperglycaemias, insulin resistance, development of type 2 diabetes and dyslipemias and given its anorexigenic role, fat oxidation stimulant, hypoglycaemic, sensitizing agent of the action of insulin on a skeletal muscle level and inhibitor of the intestinal transport of glucose by enterocytes.

### BACKGROUND OF THE INVENTION

Obesity is a serious public-health problem which has reached epidemic proportions in many developed countries (Bellanger and Bray, 2005, J The State Med Soc;157:S42-49; quiz 49). The increased food intake, unhealthy diet habits, and sedentary lifestyle in our developed countries have undoubtedly contributed to the obesity boom (Stein and Colditz et al., 2004, J. Clin. Endocrinol. Metab. 89:2522-5). Many studies have shown that the alarming increase in the prevalence of obesity and its metabolic disorders is associated to insulin resistance, dyslipemias, and eventual failure of the beta cells in the pancreas, leading inexorably to type 2 diabetes with all the consequences this disease entails (Rana et al., 2007, Diabetes Obes. Metab., 9:218-232).

Current treatments for obesity are the following: i) Xenical (Orlistat) is an inhibitor of gastrointestinal lipase which produces a moderate weight loss but has the main problem of adverse gastrointestinal effects, including colon cancer. ii) Sibutramine is a monoamine-capturing inhibitor which produces a greater weight loss than the previous treatment, but is associated to an increase in blood pressure and an increase in cardiac output. iii) Finally, use of Rimonabant, which is approved in Europe (though not by the FDA), and is an endocannabinoid receptor antagonist, but which has the problem of causing alterations in emotional behaviour such as depression, for which reason it has been withdrawn from the market.

As regards current treatments for insulin resistance, generally thiazolidinediones (TZDs) are used in combination with other therapies. The problem with these drugs is weight gain, especially when administered together with insulin. From all of this it can be gathered that an agent capable of sensitizing or increasing insulin action in addition to reducing body weight may be of great interest in these pathologies. Moreover, treatments with peripheral actions such as intestinal glucose inhibition could be of great interest in obesity and diabetes mellitus.

The discovery of leptin at the end of 1994 opened up new horizons in the study of the factors secreted by the adipocyte in regulating energy balance. This protein is mainly produced and secreted by the adipocyte proportionally to the fat mass. This protein was identified as a central nervous system modulator for appetite regulation, in addition to peripheral actions increasing fat oxidation and glucose capture by muscles. However, it was soon observed that obesity was related to high leptin levels and that endogenous leptin was not effective, for which reason the majority of obese humans and rodents were leptin-resistant, therefore this therapeutic weapon would be limited to individuals with disorders that result in leptin deficiency (a low percentage of obese people).

Since leptin is structurally similar to the cytokines of the gp-130 family and the leptin receptor (LRb) is structurally similar to the gp130 cytokine receptor known as gp-130Rbeta, the capacity of another member of the gp130 cytokine family, in particular CNTF, as a possible therapeutic agent for the treatment of obesity in order to overcome the problem of leptin resistance observed in obese individuals has been studied.

It is known that the CNTF cytokine has a possible therapeutic potential in mice suffering from obesity, insulin resistance, and a fatty liver, as it improves all these parameters (Sleeman et al., 2003, Proc Natl Acad. Sci. USA, 100:14297-14302). The anorexigenic role of CNTF (Stephens TW et al., 1995), the capacity of this cytokine to revert insulin resistance through the AMPK activation capacity (Watt et al., 2006, Nat. Med., 12:541-548) as well as its capacity to stimulate glucose capture by muscles (Steinberg et al., 2009, Diabetes, Epub January, 09, 2009) have been described, which has led to proposing this cytokine as a possible treatment for obesity and related diseases (Ahima et al., 2006, Nat. Med., 12:511-512). Nevertheless, the use of this cytokine as a therapeutic agent is limited by the low expression of the CNTFRα receptor in adipose and muscle tissue, by the high number of CNS, and by the production of anti-CNTF antibodies. Clinical trials with this cytokine in patients weighing approximately 114 kg for 84 days at doses of 1-2 micrograms/kg led to the loss of 3-4 kg. Whilst these results seemed promising, nausea appeared in patients with high CNTF doses, and they eventually gained weight due to the development of anti-CNTF antibodies (Ettinger M.P. et al., JAMA, 2003, 289:1826-32).

Therefore, there is a need in the state of the art for agents useful for the treatment of obesity which do not have the drawbacks of the gp130-based ligands known to date.

### SUMMARY OF THE INVENTION

In a first aspect, the invention is related to a compound that induces cardiotrophin 1 (CT-1) activity for the treatment of a metabolic disease, as well as to the use of a compound that induces cardiotrophin 1 (CT-1) activity for the preparation of a drug for the treatment of a metabolic disease according to the claims.

In another aspect, the invention is related to cosmetic methods which comprise administering the patient a pharmaceutically active quantity of a compound that induces cardiotrophin 1 activity according to the claims.

In another aspect, the invention is related to a composition that comprises, together or separately, a compound that induces cardiotrophin activity and an anti-diabetic compound. In successive aspects, the invention is related to a composition in accordance with the invention for the treatment of a metabolic disease, wherein the composition is administered simultaneously, separately or sequentially, as well as to the use of a composition in accordance with the invention for the preparation of a drug for the treatment of a metabolic disease, wherein the composition is administered simultaneously, separately or sequentially.

In another aspect, the invention is related to cosmetics methods that comprise administering the patient a pharmaceutically active quantity of a composition in accordance with the invention.

### BRIEF DESCRIPTION OF THE FIGURES

Figure 1: Cardiotrophin-1 reduces body weight in mice with high-fat diet-induced obesity. (S: Serum, administration vehicle of rCT-1). The results are expressed as mean ± SE. n=8 animals per group. ** p<0.01; ***p<0.001.
Figure 2: The reduction in body weight in mice with high-fat diet-induced obesity mediated by CT-1 is due, at least in part, to an anorexigenic effect. The results are expressed as mean ± SE. n=8 animals per group. * p<0.05.
Figure 3: Cardiotrophin-1 reduces body weight in mice with a normal diet (NCD). (S: Serum, administration vehicle of rCT-1). The results are expressed as mean ± SE. n=5 animals per group. ** p<0.01; ***p<0.001.
Figure 4: The reduction in body weight mediated by CT-1 is due to factors other than the anorexigenic effect (the results are expressed as mean ± SE. n=6 animals per group. *p<0.05). Pair-fed (PF) group is a group of mice fed the same daily intake as the mice treated with CT-1.
Figure 5: The acute intravenous administration of a single dose of rCT-1 (10µg) to mice C57BL/6 (3 months old) reduces baseline blood sugar levels. Levels of Blood sugar, Insulin and western blot, where AKT (P-AKT) activation is seen in muscle are taken 1 hour after administration of rCT-1. The results are expressed as mean ± SE. n=5 animals per group. *p<0,05.
Figure 6: The acute administration of a single dose of rCT-1 (10µg) prevents the increase in blood glucose after a high sugar diet. The results are expressed as mean ± SE. n=5 animals per group. *p<0,05.
Figure 7: CT-1 has direct hypoglycaemic effects in mice with pancreatic destruction (with streptozotocin). The administration of CT-1 alone decreases blood sugar levels and the effect is increased when it was administered together with insulin. The results are expressed as mean ± SE. n=5 animals per group. *p<0.05 ** p<.0.01 vs saline group. The x-axis indicates the time, in minutes, elapsed since the administration of the different treatments.
Figure 8: The hypoglycaemic effects of CT-1 is due, at least in part, to an increase in glucose consumption by muscles. The results are expressed as mean ± SE. n=5 animals per group.*p<0.05 ** p<0.01 vs saline group.
Figure 9: Glucose and insulin levels after chronic treatment with rCT-1 for 6 days in mice with high-fat diet-induced obesity. The results are expressed as mean ± SE. n=7 animals per group.*p<0.05.
Figure 10: Glucose and Insulin levels after chronic treatment with rCT-1 or after calorie restriction (PF) during 1 week. The PF (pair fed) group are mice with the same daily intake as those treated with CT-1. The results are expressed as mean ± SE. n=6 animals per group.
Figure 11: Cardiotrophin-1 inhibits intestinal glucose transport (*in vitro* treatment). The results are expressed as mean ± SE of 12 determinations in 6 different mice. **p<0.01.
Figure 12: Cardiotrophin-1 has a greater inhibitory effect of intestinal glucose transport (*in vitro* treatment) than CNTF. The results are expressed as mean ± SE of 24-35 determinations in 6 different mice. ***p<0.001
Figure 13: Cardiotrophin-1 inhibits intestinal glucose transport (acute treatment *in vivo*).
The results are expressed as mean ± SE of 30 determinations. n= 5 mice per experimental group. **p<0.01.
Figure 14: Cardiotrophin-1 inhibits intestinal glucose transport (chronic treatment *in vivo*). The results are expressed as mean ± SE of 30 determinations. n= 5 mice per experimental group. ***p<0.001.
Figure 15: Cardiotrophin-1 inhibits intestinal glucose transport (studies in Caco-2 cells). The results are expressed as mean ± SE of 42-44 determinations per group.
Figure 16: Cardiotrophin-1 inhibits leptin release in adipocytes (not CNTF) (*in vitro* experiments in primary cultures of adipocytes). The results are expressed as mean ± SE of 6 independent experiments **p<0.01; ***p<0.001 vs control; *^{b}*p<0.01; *^{c}*p<0.001 vs Insulin
Figure 17: Differential effects of CT-1 and CNTF on lipolysis. Glycerol release (measure of lipolysis) in primary rat adipocytes produced by CT-1 or CNTF in the presence or abscence of insulin during 24 hours. n = 4-8; **P*<0.05 versus control cells (100%); #*P*<0.05 versus insulin treated cells
Figure 18: Differential effects of CT-1 and CNTF on gene induction typical from brown adipose tissue in 3T3-L1 adipocytes differenciated to adipocytes. Cells were treated with CT-1 or CNTF (20 ng/ml) during 24 hours (n = 5; **P* < 0.05 versus control cells). Gene expression was measured by quantitative PCR.
Figure 19: Serum levels of IL-6 after chronic treatment with rCT-1 for 6 days.
Figure 20: Images representing histological liver slices (H&E 100X) in 3 groups of mice (C57BL/6, 4 months of age) treated with serum, CT-1 or treated with the same diet as the animals treated with CT-1 (pair-fed). In no case were inflammatory infiltrates observed.
Figure 21: rCT-1 causes a decrease in the concentration of free fatty acids (FFA) in serum and triglycerides (TG) after a high fat intake.
Figure 22: Effect of rCT-1 in the elimination of serum FFA after an intralipid injection.
Figure 23: Effect of the acute administration of rCT-1 on beta oxidation in isolated mitochondria in skeletal muscle. The data corresponds to the mean ± SE (n= ***, **P*<0.05).
Figure 24: Effect of rCT-1 on expression levels of genes involved in the oxidation of fatty acids measured by quantitative PCR.
Figure 25: Enhancing effect of rCT-1 on glucose (2- deoxiglucose) uptake by insulin in L6E9 cells (myoblasts differenciated to myotubes). Acute (1 hour) and chronic (24 hours) treatment with CT-1 increases significantly glucose uptake induced by insulin. **P*<0.05 versus control cells; #*P*<0.05 versus insulin treated cells.
Figure 26: Enhancing effect of rCT-1 on insuling signalling in L6E9 cells (myoblasts differenciated to myotubes). (A) Acute treatment with rCT-1 (15 minutes) and (B) chronic treatment with rCT-1 (10 hours and 24 hours) enhances the AKT phosphorylation induced by insulin
Figure 27: Enhancing effect of rCT-1 (acute treatment) on insulin signaling "in vivo" in muscle. 4 months of age C57BL/6 mice were injected with rCT-1 (10 µg/mouse) 30 minutes before insulin injection in cave vein. After 5 minutes the gastrocnemius muscle was extracted and homogenized. It was observed that CT-1 enhances AKT phosphorylation induced by insulin in muscle. Densitometric analysis (n=5; **P*<0.05).

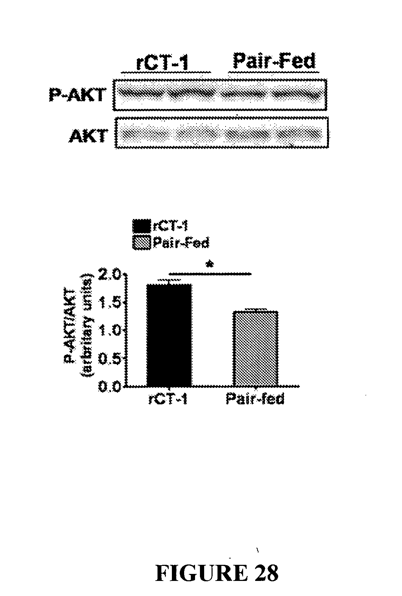

Figure 28: Enhancing effect of rCT-1 (chronic treatment) on insulin signaling "in vivo" in muscle. 4 months of age C57BL/6 mice were injected with rCT-1 (0,2 mg/kg/day) during 6 days. Mice fed with the same daily intake as the previous group and injected during 6 days with saline serum (group pair-fed) were used as a control. After 16 hours of fasting, insulin was injected in the cave vein of animals and after 5 minutes gastrocnemius muscle was extracted and homogenizes. It was observed that CT-1 enhances AKT phosphorylation induced by insulin in muscle. Densitometric analysis ((n=5; **P*<0.05)

### DETAILED DESCRIPTION OF THE INVENTION

### Medical and cosmetic uses of CT-1

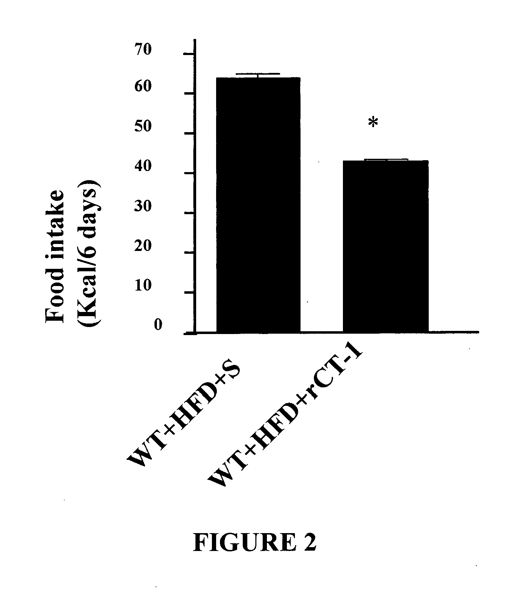

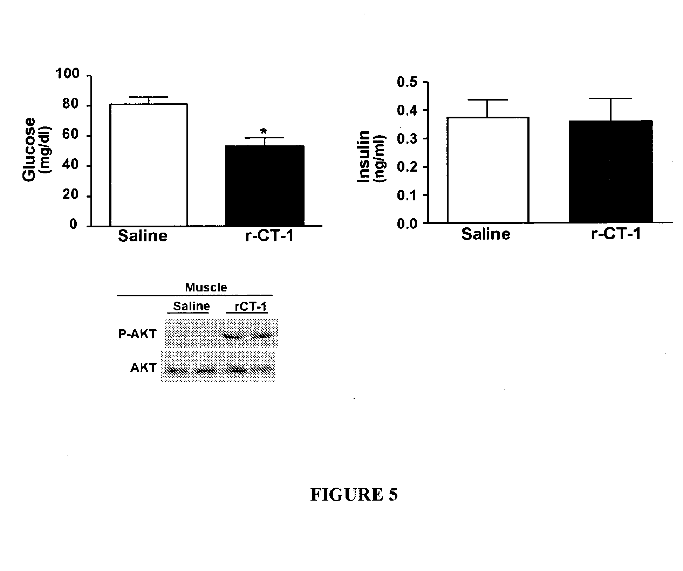

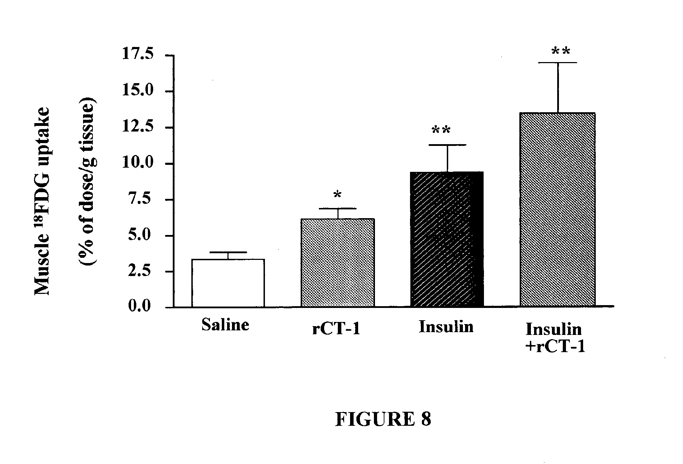

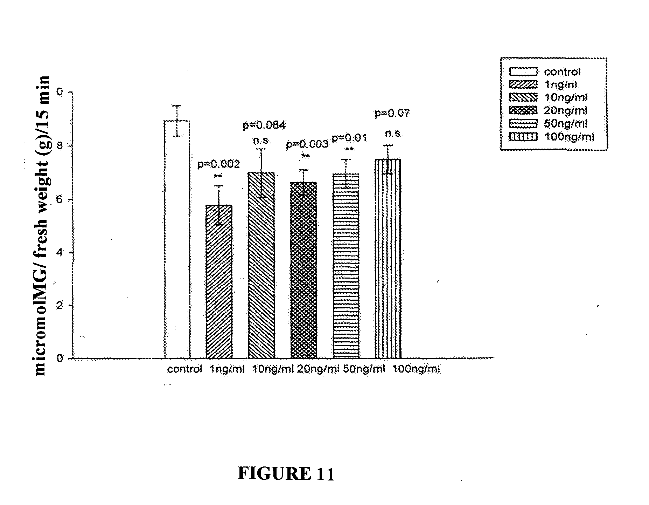

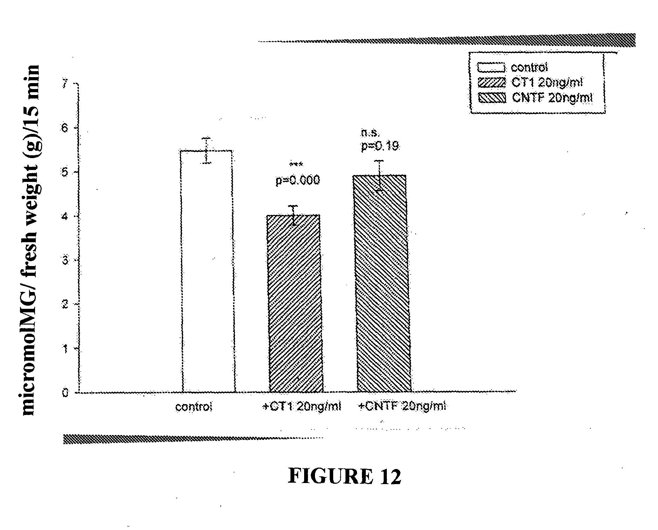

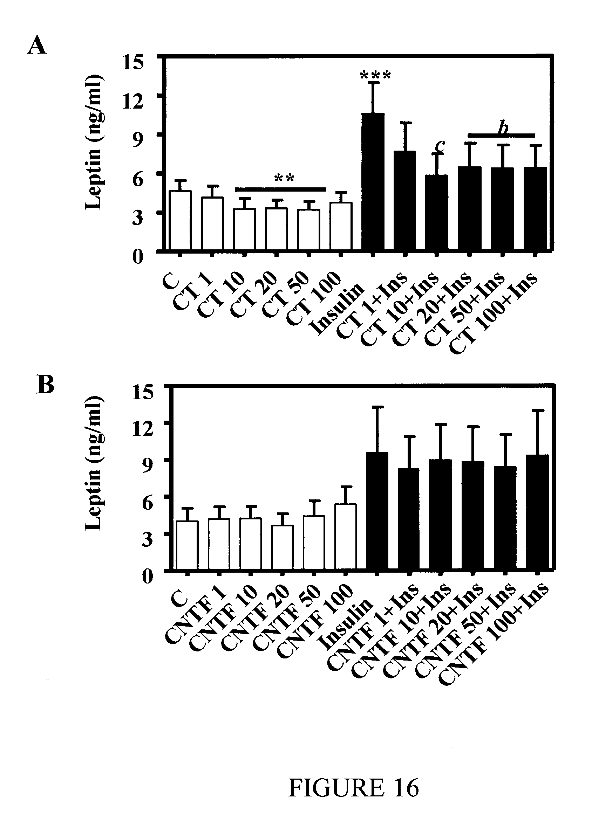

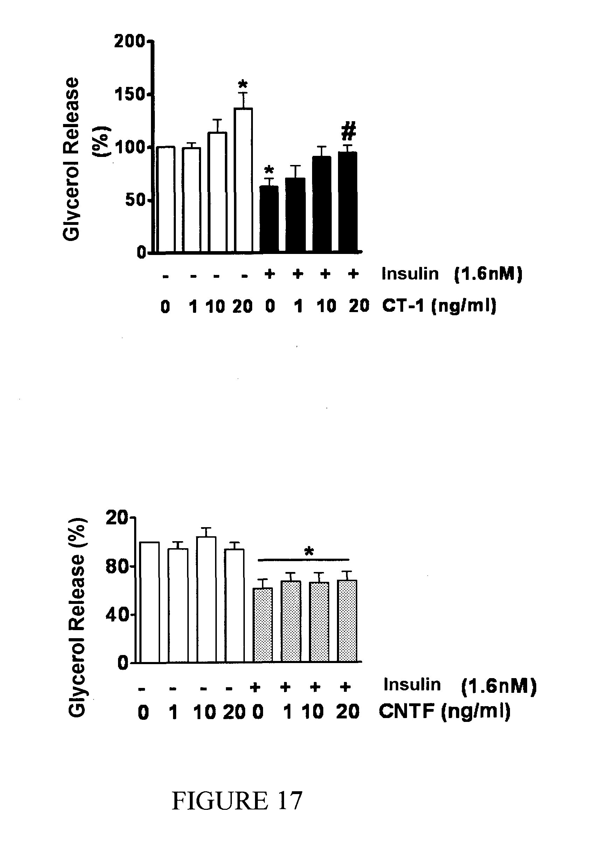

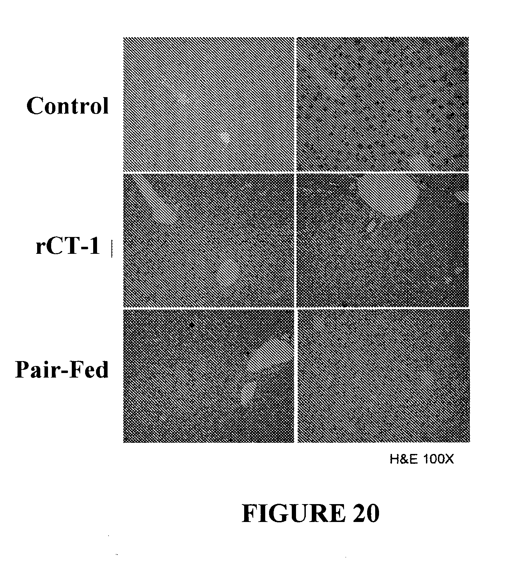

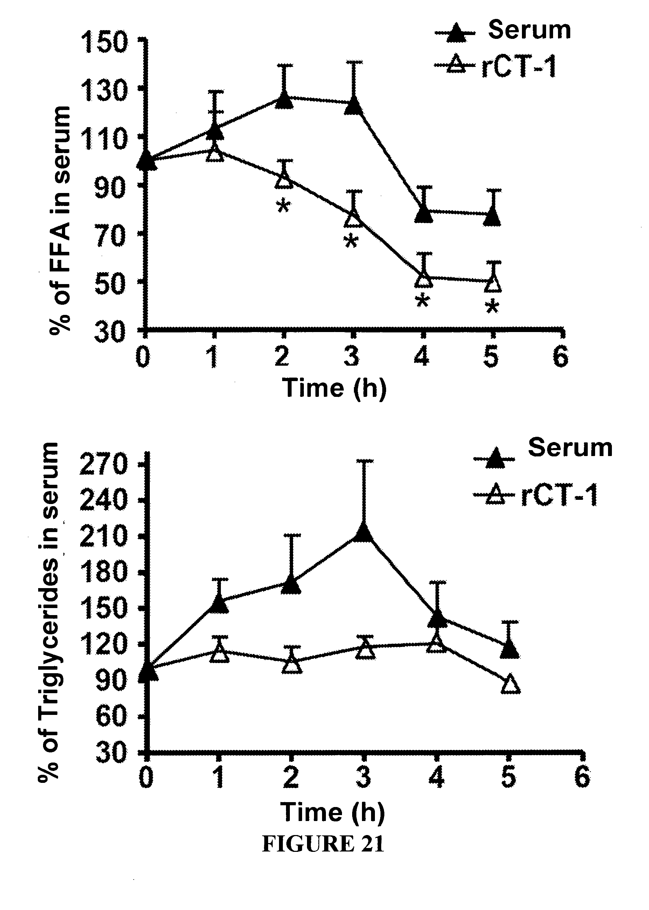

The authors of the present invention have observed that, surprisingly, CT-1 is capable of causing weight reduction in mice fed a normal diet as well as in mice fed a high-fat diet. Said effect depends, at least in part, on the anorexigenic effect of CT-1 (figure 2) as well as on the capacity of CT-1 of decreasing the increase in plasma lipids following a high-fat diet (figures 20 and 21), of decreasing blood sugar after a high sugar diet (figures 5-6), of causing an increase in glucose consumption by muscles (figure 8) and of decreasing glucose capture by the intestine (figures 11-14). Additionally, the authors of the present invention have shown that CT-1 is able to inhibit basal and insulin-stimulated leptin secretion in adipocytes, as well as to induce the release of glycerol and the inhibition of the anti-lipolytic activity of insulin (figures 16 and 17). However these effects are not observed when CNTF is used. While not adhering to any particular theory, it is thought that this differential effect might give the use of CT-1 an advantage, since said effect would be added to the action that this molecule has on muscle (stimulation of glucose capture with the consequent hypoglycaemia), causing a decrease in the glucose available for the adipocyte to turn into triglycerides.

Thus, in a first aspect, the invention is related to a compound that induces cardiotrophin 1 (CT-1) activity for the treatment of a metabolic disease. Additionally, the invention is related to the use of a compound that induces cardiotrophin-1 activity for the preparation of a drug for the treatment of a metabolic disease as well as with a method of treatment of a metabolic disease which comprises administration to a subject of a compound that induces CT-1 activity.

The term "compound that induces cardiotrophin-1 activity", as used here, is understood as any compound administration of which causes an increase in cardiotrophin-1 activity (CT-1) irrespective of whether said increase is caused by an increase in the specific activity of the pre-existing CT-1 or by an increase in the synthesis of CT-1 or analogs thereof that substantially share the same function as cardiotrophin. Thus, in a preferred embodiment, the agent that induces CT-1 activity is CT-1 itself. "CT-1" is understood as the protein defined by the sequence described in the UniProtKB database by access number CTF1_HUMAN or Q16619 in version 62 dated 16-DEC-2008, corresponding to isoform 1 of human cardiotrophin, or access number Q5U5Y7 in its version 1 dated 20.01.2009, corresponding to isoform 2 of human cardiotrophin, as well as orthologues in other species, such as mouse (sequences defined in the UniProtKB database with access numbers NP_031821 or Q60753 in version 50 dated 16.12.2008, and NP_942155 or P83714 in version 42 dated 16.12.2008), rat (sequences defined in the UniProtKB database with access numbers NP_058825 or Q63086 in version 50 dated 04.11.2008, and NP_001129272 or Q6R2R3 in version 30 dated 20.01.2009) and chimpanzee (sequence defined in the UniProtKB database with access number NP_001009112 or Q6R2R2 in version 30 dated 04.11.2008).

In another embodiment, the agent that induces CT-1 activity is a functionally equivalent variant of CT-1. The expression "functionally equivalent variant of CT-1", as used here, is understood as any molecule which shares with CT-1 one or more of the functions described in the present invention associated to CT-1, both *in vitro* and *in vivo* and which has a minimum of 90% identity in the amino acid sequence. Thus, variants of CT-1 suitable for their use in the present invention are derived from the aforementioned sequences by insertion, substitution or deletion of one or more amino acids and include natural alleles, variants resulting from alternative processing and secreted and truncated forms which appear naturally. The variants of CT-1 show an identity of sequence with CT-1 of at least 90%, at least 91%, at least 92%, at least 93%, at least 94%, at least 95%, at least 96%, at least 97%, at least 98% or at least 99%. The degree of identity is determined using methods very well known to those skilled in the art. The identity between two amino acid sequences is preferably determined using the BLASTP algorithm [BLASTManual, Altschul, S., et al., NCBI NLM NIH Bethesda, Md. 20894, Altschul, S., et al., J. Mol. Biol. 215: 403-410 (1990)] preferably using the default parameters. On the other hand, the variants of CT-1 envisaged show at least one of the functions of CT-1 such as, without limitation:
- The capacity of reducing body weight in mice both when they have been fed a normal diet and when obesity has been induced by a high-fat diet. Methods for determining the capacity of a compound of causing a decrease in body weight have been described extensively in the state of the art and include methods such as those described in example 1 of the present invention. In the case of the determination of weight loss in mice fed a high-fat diet, any high-fat diet known in the state of the art can be used such as the diets called D12450B or LFD which provides 10% of kilocalories in the form of fat, diet D12451 or (HFD) which provides 45% of kilocalories in the form of fats or diet D12492 (VHFD) which provides 60% of kilocalories in the form of fat.
- the capacity of inducing an anorexigenic effect, which can be determined using the method described in example 1 of the present invention or using the method described in WO2007093363.
- the capacity of decreasing the respiratory quotient, defined as the ratio between the values of V_{O2} and V_{CO2}. This parameter can be determined using any method known in the state of the art.
- the capacity of preventing the increase of lipidemia (both regarding triglycerides and free fatty acids) after a high-fat diet, for which standard methods are used to determine the concentration of fatty acids and triglycerides in plasma, preferably through the use of commercial kits.
- the direct hypoglycaemic capacity, or after a high sugar diet or after pancreatic destruction, which can be determined by the use of commercial glucose detection kits. Pancreatic destruction can be caused by streptozotocin as described in the example 3 of the present invention or by the use of proinflammatory cytokines (for example, the cocktail formed by IL-1b, TNF-a and IFN-γ described by Choi et al. in Transplant Immunol., 2004, 13:43-53.
- the capacity of causing an increase in glucose capture by muscles, wherein said activity is determined using the method described in example 3 of the present application based on the capacity of the gastronomic muscles of absorbing an analog marked with glucose or using non-isotopic methods such as that described by Ueyama et al (Biol. Signals Recept 2000; 9:267-274).
- the capacity of inhibiting the effects of insulin on glucose capture and the release of leptin by adipose tissue, for which both primary cultures of adipocytes and fibroblastic cell lines which have been treated to promote their differentiation in adipocytes can be used. The release of glucose by the adipocytes can be carried out through the use of commercial glucose detection kits. The determination of leptin can be carried out through immunoassays (ELISA, radioimmunoassay, etc) using commercial kits.

In another embodiment, the agent capable of inducing CT-1 activity is a polynucleotide that codes for CT-1 or a functionally equivalent variant thereof.

The term "polynucleotide", as used in the present invention, relates to a polymeric form of nucleotides of any length and formed by ribonucleotides and/or deoxyribonucleotides. The term includes both single-chain and double-chain polynucleotides, as well as modified polynucleotides (methylated, protected and such like).

Polynucleotides suitable for use as agents capable of inducing CT-1 activity include, without limitation, the polynucleotides the sequences of which correspond to those described in the GenEMBL database with access numbers BC064416 in version 9 with date 15.10.2008, corresponding to variant 1 of the transcript of human cardiotrophin, BC036787 in version 9 with date 12.08.2009, corresponding to variant 1 of the transcript of human cardiotrophin, the sequence described in the GenEMBL database with access number D78591, in its version 4 of 12.01.2009, corresponding to the polynucleotide that codes for cardiotrophin 1 of Rattus norvegicus (rat), the sequence described in the GenEMBL database with access number AY518205, in its version 3 of 12.01.2009, corresponding to the polynucleotide that codes for cardiotrophin 2 of *Rattus norvegicus* (rat), the sequence described in the GenEMBL database with access number U18366, in its version 4 of 12.01.2009, corresponding to the polynucleotide that codes for cardiotrophin 1 of *Mus musculus* (mouse), the sequence described in the GenEMBL database with access number AB125661, in its version 2 of 12.01.2009, corresponding to the polynucleotide that codes for cardiotrophin 2 of *Mus musculus* (mouse).

Alternatively, the agents capable of inducing CT-1 activity include functionally equivalent variants of the polynucleotides previously defined by means of their specific sequences. "Functionally equivalent polynucleotide" is understood, in the context of the present invention, as all those polynucleotides capable of coding for a polypeptide with CT-1 activity, as previously defined, and which result from the aforementioned polynucleotides by means of the insertion, deletion or substitution of one or several nucleotides with respect to the aforementioned sequences. Preferably, the variant polynucleotides of the present invention are polynucleotides the sequence of which allows them to hybridize in highly restrictive conditions with the aforementioned polynucleotides. Typical conditions of highly restrictive hybridization include incubation in 6 X SSC (1 X SSC: 0.15 M NaCl, 0.015 M sodium citrate) and 40% formamide at 42 °C for 141 hours, followed by one or several cycles of washing using 0.5 X SSC, 0.1% SDS at 60°C. Alternatively, highly restrictive conditions include those which comprise hybridization at an approximate temperature of 50°-55° C in 6X SSC and a washing final at a temperature of 68° C in 1-3 X SSC. The moderate restrictive conditions comprise hybridization at a temperature of approximately 50° C until 65° C in 0.2 or 0.3 M NaCl, followed by washing at approximately 50° C until 55° C in 0.2X SSC, 0.1% SDS (sodium dodecyl sulfate).

Preferably, when the agent capable of inducing CT-1 activity is a polynucleotide, this is operationally associated to a regulatory region of the expression. The regulatory sequences used in the present invention can be nuclear promoter sequences or, alternatively, enhancer sequences and/or other regulatory sequences that increase the expression of the heterologous nucleic acid sequence. The promoter can be constitutive or inducible. If one wants a constant expression of the heterologous nucleic acid sequence, then a constitutive promoter is used. Examples of well-known constitutive promoters include the cytomegalovirus (CMV) immediate-early promoter, Rous sarcoma virus promoter and such like. Many other examples of constitutive promoters are well known in the state of the art and can be used in the practice of the invention. If the controlled expression of the heterologous nucleic acid sequence is desired, then an inducible promoter should be used. In a non-induced state, the inducible promoter is "silent". "Silent" means that in the absence of an inducer little or no expression of the heterologous nucleic acid sequence is detected; in the presence of an inducer, however, the expression occurs of the heterologous nucleic acid sequence. Frequently, it is possible to control the expression level by varying the inducer concentration. Controlling the expression, for example varying the concentration of the inducer so that an inducible promoter is more strongly or more weakly stimulated, it is possible to affect the concentration of the transcript product of the heterologous nucleic acid sequence. In the case that the heterologous nucleic acid sequence codes for a gene, it is possible to control the quantity of synthesized protein. In this way, it is possible to vary the concentration of the therapeutic product. Examples of well-known inducible promoters are: an estrogen or androgen promoter, a metallothionein promoter, or a promoter that responds to ecdysone. Many other examples are well known in the state of the art and can be used in the practice of the invention. In addition to the constitutive and inducible promoters (which usually function in a great variety of cells or tissues), specific tissue promoters can be used to achieve expression of the heterologous sequence of specific nucleic acid in cells or tissues. Well-known examples of specific tissue promoters include different specific muscle promoters including: skeletal α-actin promoter, cardiac actin promoter, skeletal troponin C promoter, cardiac troponin C promoter and of slow contraction, and the creatine kinase promoter/enhancer. There are specific muscle promoters which are well known in the state of the art and which can be used in the practice of the invention (for a review of specific muscle promoters, see Miller et al., (1993) Bioessays 15: 191-196).

In another embodiment, the agent capable of inducing CT-1 activity is a vector that comprises a polynucleotide as previously defined, i.e. that codes for CT-1 or a functionally equivalent variant thereof. Suitable vectors for the insertion of said polynucleotides are vectors derived from expression vectors in prokaryotes such as pUC18, pUC19, pBluescript and their derivatives, mp18, mp19, pBR322, pMB9, ColE1, pCR1 , RP4, phages and "shuttle" vectors such as pSA3 and pAT28, yeast expression vectors such as vectors of the 2 micron plasmid type, integration plasmids, YEP vectors, centromeric plasmids and such like, expression vectors in insect cells such as the vectors of the pAC series and of the pVL series, expression vectors in plants such as vectors of the pIBI, pEarleyGate, pAVA, pCAMBIA, pGSA, pGWB, pMDC, pMY, pORE series and such like and expression vectors in eukaryote cells either based on viral vectors (adenovirus, virus associated to adenovirus as well as retrovirus and, in particular, lentivirus) as well as non-viral vectors such as pSilencer 4.1-CMV (Ambion), pcDNA3, pcDNA3.1/hyg pHCMV/Zeo, pCR3.1, pEFl/His, pIND/GS, pRc/HCMV2, pSV40/Zeo2, pTRACER-HCMV, pUB6/V5-His, pVAX1, pZeoSV2, pCI, pSVL and pKSV-10, pBPV-1, pML2d and pTDT1.

In another embodiment, the agent capable of causing an increase in CT-1 activity is a cell capable of secreting cardiotrophin-1 or a functionally equivalent variant thereof into the medium. Suitable cells for the expression of cardiotrophin-1 or of the functionally equivalent variant thereof include, without limitation, cardiomyocytes, adipocytes, endothelial cells, epithelial cells, lymphocytes (B and T cells), mastocytes, eosinophils, cells of the vascular intima, primary culture of cells isolated from different organs, preferably from cells isolated from islets of Langerhans, hepatocytes, leukocytes, including mononuclear, mesenchymal, umbilical cord or adult leukocytes (from the skin, lungs, kidney and liver), osteoclasts, chondrocytes and other connective tissue cells. Established cell lines such as Jurkat t cells, NIH-3T3, CHO, Cos, VERO, BHK, HeLa, COS, MDCK, 293, 3T3 cells, C2C12 myoblasts and W138 cells are also suitable.

Those skilled in the art will appreciate that cells capable of secreting cardiotrophin-1 or a functionally equivalent variant thereof into the medium can be found forming microparticles or microcapsules so that the cells have a greater useful life before being used in patients. Suitable materials for forming the microparticles that are an object of the invention include any biocompatible polymeric material that allows the continuous secretion of therapeutic products, acting as cell support. Thus, said biocompatible polymeric material can be, for example, thermoplastic polymers or hydrogel polymers. Thermoplastic polymers include acrylic acid, acrylamide, 2-aminoethyl methacrylate, poly(tetrafluoroethylene-cohexafluoropropylene), methacrylic-(7-cumaroxy) ethyl ester acid, N-isopropyl acrylamide, polyacrylic acid, polyacrylamide, polyamidoamine, poly(amino)-p-xylylene, poly(chloroethyl vinyl ether), polycaprolactone, poly(caprolactone-co-trimethylene carbonate), poly(carbonate urea) urethane, poly(carbonate) urethane, polyethylene, polyethylene copolymer and acrylamide, polyethylene glycol, polyethylene glycol methacrylate, poly(ethylene terephthalate), poly(4-hydroxybutyl acrylate), poly(hydroxyethyl methacrylate), poly(N-2-hydroxypropyl methacrylate), poly(lactic acid-glycolic acid), poly(L lactic acid), poly(gamma-methyl, L-glutamate), poly(methylmethacrylate), poly(propylene fumarate), poly(propylene oxide), polypyrrole, polystyrene, poly(tetrafluoroethylene), polyurethane, polyvinyl alcohol, ultra-high molecular weight polyethylene, 6-(p-vinybenzamide)-hexanoic acid and N-p-vinylbenzyl-D-maltonamide and copolymers which contain more than one of said polymers. The polymers of hydrogel type include natural materials of alginate, agarose, collagen, starch, hyaluronic acid, bovine serum albumin, cellulose and its derivatives, pectin, chondroitin sulfate, fibrin and fibroin type as well as synthetic hydrogels such as sefarose and sefadex.

It is known in the state of the art that some of said polymers are unstable and tend to lose their gel nature, in addition to being relatively porous, which means that antibodies may access their interior and damage the cells. For this reason, optionally, the microparticle of the invention can be surrounded by a semi-permeable membrane which gives stability to the particles, forming a barrier impermeable to antibodies. Semipermeable membrane means a membrane that allows the entry of all those solutes necessary for cell viability and allows the exit of therapeutic proteins produced by the cells contained in the microparticle, but which is substantially impermeable to antibodies, so that the cells are protected from the immune response produced by the organism housing the microparticle. Suitable materials for forming the semipermeable membrane are materials insoluble in biological fluids, preferably polyamino acids, such as, for example poly-L-lysine, poly-L-ornithine, poly-L-arginine, poly-L-asparagine, poly-L-aspartic, poly benzyl-L-aspartate, poly-S-benzyl-L-cysteine, poly-gamma-benzyl-L-glutamate, poly-S-CBZ-L-cysteine, poly- ε-CBZ-D-lysine, poly-δ-CBZ-DL-ornithine, poly-O-CBZ-L-serine, poly-O-CBZ-D-thyrosine, poly(γ-ethyl-L-glutamate), poly-D-glutamic, polyglycine, poly-γ-N-hexyl L-glutamate, poly-L-histidine, poly(α,β-[N-(2-hydroxyethyl)-DL-aspartamide]), poly-L-hydroxyproline, poly (α,β-[N-(3-hydroxypropyl)-DL-aspartamide]), poly-L-isoleucine, poly-L-leucine, poly-D-lysine, poly-L-phenylalanine, poly-L-proline, poly-L-serine, poly-L-threonine, poly-DL-tryptophan, poly-D-thyrosine or a combination thereof.

The term "metabolic disease", as used here, is understood as all types of disorders that lead to errors and imbalances in the metabolism as well as to metabolic processes taking place in a sub-optimal form. The expression also relates to disorders that can be treated through metabolism modulation, although the disease in itself may not have been caused by a metabolic disorder. In a preferred embodiment, the metabolic disease is selected from the set of obesity, hyperglycaemias, insulin resistance, type 2 diabetes, and dyslipemias.

The term "obesity", as used in the present invention, relates to the definition of obesity provided by the WHO based on the body mass index (BMI), which consists of the ratio between the weight of a person (in kg) and the square of their height in metres. According to this criteria, a BMI lower than 18.5 kg/m² is considered as insufficient weight or thinness, a BMI of 18.5-24.9 kg/m² is considered a normal weight, a BMI of 25.0-29.9 kg/m² is considered grade 1 of overweight, a BMI of 30.0-39.0 kg/m² is considered a grade 2 of overweight and a BMI greater than or equal to 40.0 kg/m² is considered morbid obesity. Alternatively, there are alternative methods for defining the an individual's degree of obesity, such as the diameter of the waist measured at the midpoint between the lower limit of the ribs and the upper limit of the pelvis (in cm), the thickness of skin folds, and bioimpedance, based on the principle that a lean mass transmits electricity better than a fatty mass.

The term "hyperglycaemia", as used in the present invention, relates to a state where abnormally high blood glucose levels appear in relation to the fasting baseline levels. In particular, hyperglycaemia is understood to take place when fasting blood glucose levels are consistently higher than 126 mg/dL, the postprandial glucose levels are higher than 140 mg/dL, and/or the glucose levels in venous plasma 2 hours after administration of a dose of glucose of 1.75 grams for each kilogram of body weight is over 200 mg/dL.

The term "insulin resistance", as used in the present invention, relates to a disorder wherein the cells do not respond correctly to insulin. As a result, the body produces more insulin in response to high blood glucose levels. Patients with insulin resistance frequently display high glucose levels and high circulating insulin levels. Insulin resistance is frequently linked to obesity, hypertension, and hyperlipidemia. Additionally, insulin resistance frequently appears in patients with type 2 diabetes.

The term "type 2 diabetes", as used in the present invention, relates to a disease characterized by an inappropriate increase in blood glucose levels, which generates chronic complications as it affects large and small vessels and nerves. The underlying disorder in this disease is the difficulty for insulin action (in the form of a loss of tissue sensitivity to this hormone), which is called insulin resistance, and an inadequate secretion of insulin by the cells responsible for their production in the pancreas. In addition to increasing glucose concentration, faulty insulin action frequently translates into an increase in cholesterol and/or triglyceride levels.

The term "dyslipemia", as used in the present invention, relates to any pathological condition characterized by a disorder in the lipid metabolism, with a consequent disorder in lipid concentration (cholesterol, triglycerides and such like) and lipoproteins (high density lipoproteins) in the blood. Dyslipemias that can be treated with the methods of the present invention include, without limitation, hypercholesterolemia, hypertriglyceridemia, hyperlipoproteinemia of type I, IIa, IIb, III, IV, V, hyperchylomicronemia, combined hyperlipidemia, etc.

The compositions of the invention are useful both for the treatment of morbid obesity and for the treatment of grade 1 or grade 2 of overweight, in which case the methods of the invention have a cosmetic purpose. Therefore, in another aspect, the invention is related to a cosmetic method for the treatment of obesity which comprises administering to a patient a compound that induces cardiotrophin 1 activity. In this embodiment, the patients with excess weight in the form of fat and who can be treated by the cosmetic method of the present invention are identified visually or by having a BMI higher than or equal to 25 kg/m², preferably between 25 and 30. These individuals are considered obese, and needing weight control for cosmetic reasons.

The different embodiments of the cosmetic method in accordance with the invention as regards agents capable of inducing cardiotrophin-1 activity have been described previously in the context of the methods of treatment of obesity and other metabolic diseases.

The compounds of the invention can be administered both for acute and for chronic treatment. The expression "chronic administration", as used in the present invention, relates to a method of administration wherein the compound is administered to the patient continuously for extended periods of time in order to maintain the therapeutic form during said period. Forms of chronic administration include the administration of multiple doses of the compound daily, twice daily, thrice daily or with more or less frequency. Chronic administration can be carried out by means of different intravenous injections administered periodically throughout a single day. Alternatively, chronic administration involves the administration in the form of bolus or by continuous transfusion which can be carried out daily, every two days, every 3 to 15 days, every 10 days or more. Typically, chronic administration is maintained for at least 72 hours, at least 96 hours, at least 120 hours, at least 144 hours, at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 4 months, at least 5 months, at least 6 months, at least 9 months, at least one year, at least 2 years or more.

The expression "acute administration", as used in the present invention, relates to a method of administration wherein the patient is exposed to a single dose of the compound or several doses but over a reduced time period, such as, for example, 1, 2, 4, 6, 8, 12 or 24 hours or 2, 3, or 4 days.

Those skilled in the art will appreciate that the effective therapeutic quantity and/or formulation of the active compound shall be carried out depending on the type of administration. "Therapeutically effective quantity", as used here, is understood as the quantity of compound that makes it possible to totally or partially alleviate the symptoms associated with a metabolic disease or which prevents the progression or worsening the symptoms or which prevents the appearance of the disease in a subject at risk from suffering the disease.

If chronic administration of the compound of the invention is desired, it can be administered in a sustained release composition such as that disclosed in documents US5672659, US5595760, US5821221, US5916883 and WO9938536. Otherwise, if acute administration is desired, a treatment with immediate release will be preferred. Irrespective of the type of administration, the quantity of the dose and the interval can be individually adjusted to provide plasma levels of the compounds sufficient for maintaining the therapeutic effect. Someone with normal experience in the state of the art will be capable of optimizing the therapeutically effective local doses without too much experimentation.

Administration of the compounds of the invention requires their formulation in pharmaceutical compositions, which constitute another aspect of the invention. Useful pharmaceutical compositions in the practice of the method of the invention include a therapeutically effective quantity of an active agent, and pharmaceutically acceptable carrier. The term "pharmaceutically acceptable" means approved by a regulatory agency of the Federal government or of a state or included in the USA Pharmacopoeia or another generally recognized pharmacopoeia, for use in animals, and more particularly in humans. The term "carrier" relates to a diluent, coadjuvent, excipient, or vehicle whereby the therapeutic compound is administered. Said pharmaceutical carriers can be sterile liquids, such as water and oils, including petroleum, animal or plant origin or synthetic oils, such a peanut oil, soy oil, mineral oil, sesame oil and such like. Suitable pharmaceutical excipients include starch, glucose, lactose, sucrose, gelatine, malt, rice, flour, chalk, silica gel, sodium stearate, glycerol monostearate, talc, sodium chloride, powdered skimmed milk, glycerol, propylene glycol, water, ethanol and such like. The composition, if desired, can also contain smaller quantities of wetting or emulsifying agents, or pH buffering agents. These compositions may take the form of solutions, suspensions, tablets, pills, capsules, powder, prolonged release formulations and such like. The composition may be formulated as a suppository, with binders and traditional carriers such as triglycerides. The oral formulation can include standard carriers such as pharmaceutical types of mannitol, lactose, starch, magnesium stearate, sodium saccharin, cellulose, magnesium carbonate, etc. Examples of suitable pharmaceutical carriers are described in "Remington's Pharmaceutical Sciences" by E.W. Martin.

The composition can be formulated in accordance with routine procedures as a pharmaceutical composition adapted for intravenous, subcutaneous, or intramuscular administration to human beings. When necessary, the composition may also include a solubilising agent and a local anaesthetic such as lidocaine to relieve pain at the injection site. When the composition is going to be administered by infiltration, it can be dispensed with an infiltration bottle which contains water or saline solution of pharmaceutical quality. When the composition is administered by injection, a water vial can be provided for injection or sterile saline solution, so that the ingredients can be mixed before administration.

The quantity of CT-1 activity inducing compound that will be effective in the treatment of metabolic disease can be determined by standard clinical techniques based on the present description. Furthermore, *in vitro* tests can also be optionally used to help identify optimum dosage ranges. The precise dose to use in the formulation will depend on the administration route, and the severity of the condition, and it should be decided at the doctor's judgement and depending on each patient's circumstances. However, the dosage ranges suitable for intravenous administration are generally 50-5000 micrograms of active compound per kilogram of body weight. The dosage ranges suitable for intranasal administration are generally approximately of 0.01 pg/kg of body weight to 1 mg/kg of body weight. The effective doses can be extrapolated from a pair of response curves to doses derived from model *in vitro* assay systems or in animals.

For systemic administration, a therapeutically effective dose can be initially estimated from *in vitro* assays. For example, a dose can be formulated in animal models to achieve a circulating concentration range which includes the IC50 which has been determined in cell culture. Said information can be used to precisely determine useful doses in humans. The initial doses can also be estimated from *in vivo* data, e.g. animal models, using techniques well known in the state of the art. Someone with normal experience in the state of the art can easily optimize administration to humans based on the data in animals.

Likewise, the invention envisages the combined use of the agent capable of inducing CT-1 activity with one or several compounds capable of reducing the availability of nutrients in the organism, which have an anorexigenic effect or which have an anti-obesity effect. Thus, the metabolic disease treatment methods in accordance with the invention envisage the joint, sequential or separate administration of the compounds with capacity for inducing CT-1 activity with one or several compounds selected from the group of amylin, amylin agonists, calcitonin salmon, cholecystokinin or an agonist thereof, leptin (OB protein), exendin or an exendin analog, GLP1 or an agonist thereof, polypeptides (PYY) or agonists thereof, agents that affect neurotransmitters or neuronal ion channels such as anti-depressants, noradrenaline capture inhibitors, serotinin 2c receptor agonists, some dopaminergic antagonists, cannabinoid 1 receptor agonists, agents which modulate the route of leptin/insulin in the CNS including leptin analogs, promoters of leptin transport or promoters of the leptin receptor, CNTF, neuropeptide Y, antagonists of the peptide related to agouti, proopiomelanocortin, cocaine, amphetamine, alpha-melanocyte stimulating hormone, melanocortin-4 receptor agonists, agents which affect insulin activity/metabolism as protein tyrosine phosphate 1 beta inhibitors, antagonists of the receptor activated by the peroxisome 7 proliferator, bromocriptine, somatostatin agonists, agents that increase the metabolic rate at rest (agonists of the uncoupling protein, agonists of the thyroid hormone receptor) as well as other agents of different type such as melanin, analogs of phytostanol, functional oils, fatty acid synthesis inhibitors, carboxypeptidase inhibitors, intestinal lipase inhibitors and such like.

### Compositions of the invention

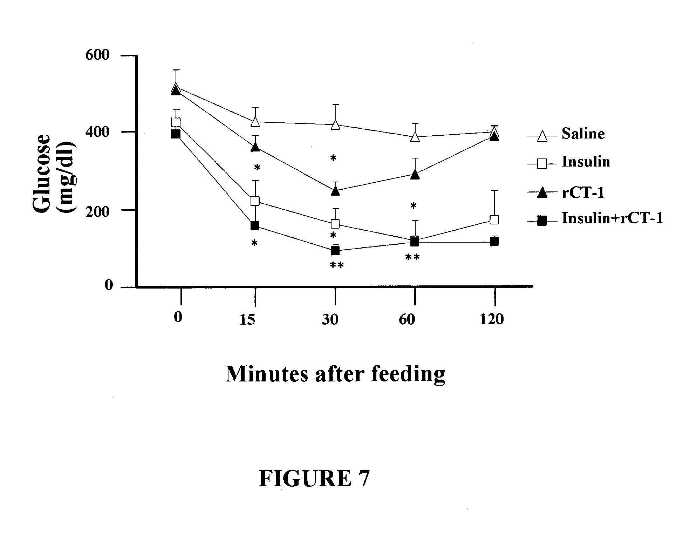

The authors of the present invention have observed that, surprisingly, the joint administration of CT-1 and insulin has hypoglycaemic effects on models of type 1 diabetes and on glucose capture by muscles which are higher than those observed by the administration of each component separately. Thus, figure 7 shows how the treatment of mice subjected to the experimental destruction of beta pancreatic cells with streptozotocin with a combination of CT-1 and insulin results in a hypoglycaemic effect higher than that observed after administration of each one of the components separately. Additionally, figure 8 shows how the combination of CT-1 and insulin causes an increase in glucose capture by muscles higher than that observed with any of the compounds administered separately.

Therefore, in another aspect, the invention is related to a composition (hereinafter, composition of the invention) which comprises, together or separately, a compound that induces cardiotrophin activity and an anti-diabetic compound.

The expression "compound that induces cardiotrophin activity" has been described previously in the context of the methods of treatment of metabolic diseases and comprises, as in the first method of the invention, a compound selected from the group of
(i) Cardiotrophin-1 (CT-1),
(ii) a functionally equivalent variant of CT-1,
(iii) a polynucleotide that codes for CT-1 or a functionally equivalent variant thereof,
(iv) a vector which comprises a polynucleotide according to (iii) and
(v) a cell capable of secreting into the medium cardiotrophin-1 or a functionally equivalent variant thereof.
wherein the different compounds have been described in detail previously.

The expression "anti-diabetic compound", as used in the present invention, is understood as an agent which, irrespective of its action mechanism, is capable of at least partially compensating for the symptoms of diabetes, including hyperglycaemia. In a preferred embodiment, the anti-diabetic compound is a compound that induces insulin activity or a compound that induces hypoglycaemic activity of insulin or sensitizing agent to insulin activity.

The anti-diabetic compounds capable of inducing insulin activity include, without limitation,
(i) insulin,
(ii) a functionally equivalent variant of insulin
(iii) a polynucleotide that codes for insulin or a functionally equivalent variant thereof
(iv) a vector that comprises a polynucleotide according to (iii)
(v) a cell capable of secreting into the medium insulin or a functionally equivalent variant thereof.

The term "insulin", as used in the present invention, relates to insulin of any species (primate, rodent or rabbit), but preferably of human origin and more preferably insulin of native sequence, which comprises a polypeptide which has the same sequence of amino acids as the insulin derived from nature. Said insulin polypeptides of native sequence can be isolated from nature or can be produced by recombinant and/or synthetic medium. The term "native sequence insulin" specifically covers the truncated or secreted forms and allelic variants that occur naturally. In an embodiment of the invention, the native sequence human insulin is a mature native sequence insulin or of a complete length which comprises an alpha or A chain, corresponding to amino acids 90 to 110 of the sequence of human preproinsulin (access number P01308 in the UniProtKB database in version 126 with date 20.01.2009) and a beta or B chain, corresponding to amino acids 25 to 54 (access number P01308 in the NCBI database in version 126 with date 20.01.2009).

"Functionally equivalent variant of insulin", as used in the present invention, is understood as all those polypeptides resulting from the elimination, insertion or modification of at least one amino acid with respect to the insulin sequence and which substantially maintains the same properties as the insulin it comes from. Insulin activity can be determined by methods widely known by those skilled in the art such as normoglycaemic clamping or the measurement of glycosylated proteins in serum (Bunn et al., Diabetes, 1981, 30:613-617).

Functionally equivalent variants of insulin include, without limitation, the des-pentapeptide (B26-B30)-Phe^{B21}-α-carboxamide]insulin, Asp^{B10} insulin (disclosed in US4992417), Lys^{B28}-Pro^{B29} insulin Lys^{B28}-Pro^{B29} and the hexameric variant thereof (disclosed in US5474978 and US5514646), formulations of insulin and protamine (US5650486), acylated Lys^{B28}-Pro^{B29} insulin (US5922675), and compositions of stabilized insulin such as those disclosed in US5952297, US6034054 and US6211144, superactive analogs of insulin, monomeric insulins, hepatospecific insulins, insulin lispro (Humalog®), insulin lispro formulated with insulin lispro protamine (marketed as Humalog ®50/50^{™}, Humalog® 75/25^{™}), NPH insulin or insulin isophane human (marketed as Humulin®), regular insulin, NPH insulin combined with regular insulin (US5547929), insulin zinc, insulin glargine, glulisine (APidra), insulin Aspart (Novomix), insulin detemir (levemir), biota, LP-100, novarapid, insulin zinc suspension (slow and ultraslow), GLP-1 (1-36) amide, GLP-1 (73-7) (insulinotropin disclosed in US5614492), LY-315902 (Lilly), GLP-I (7-36)-NH2), AL-401 (Autoimmune), compositions such as those disclosed in US4579730, US4849405, US4963526, US5642868, US5763396, US5824638, US5843866, US6153632, US6191105, and WO 85/05029.

Additionally, functionally equivalent variants of insulin include polypeptides that show at least approximately 80% of identity of amino acid sequence with the sequence of amino acids of: (a) residues 1 to 21 of the high-fat diet A chain in combination with residues 1 to 30 of the high-fat diet B chain, or (b) another fragment specifically derived from said sequences. Furthermore, the secondary structure of the insulin maintained by disulfide bonds between cysteine residues A6-Al1, A7-B7 and A20-B 19 seems necessary for the activity, for which reason the functionally equivalent variants preferably preserve said secondary structure as much as possible.

Functionally equivalent variants of insulin include, for example, the polypeptides wherein one or more amino acid residues are added, or deleted, at the N- and/or C-terminal ends, as well as within one or more of the internal domains, of the A and B chains of insulin. Normally, a polypeptide variant of insulin will have at least approximately an identity of amino acids sequence with the sequence formed by residues 1 to 21 of the A chain of the high-fat diet in combination with residues 1 to 30 of the B chain of the high-fat diet or with another fragment specifically derived from said sequences of amino acids of at least 80%, 82%, 83%, 84%, 85%, 86%, 87%, 88%, 89%, 90%, 91%, 92%, 93%, 94%, 95%, 96%, 97%, 98% or 99% of identity of amino acids sequence. The polypeptide variants will have a sequence length of at least 15, 16, 17, 18, 19, 20, 21, 22, 23, 24, 25, 26, 27, 28, 29, 30, 31, 32, 33, 34 or 35 of the A chain and at least 25, 26, 27, 28, 29, 30, 31, 32, 33, 34, 35, 36, 27, 38, 39, 40, 41, 42, 43, 44, 45, 46, 47, 48, 49 or 50 residues of the B chain.

Polynucleotides which code for insulin include both the polynucleotide that codes for human insulin as well as the precursors thereof (preproinsulin and proinsulin), as well as the orthologs of other species.

"Polynucleotide that codes for a functionally equivalent variant of insulin" indicates (a) a nucleic acid that codes for a polypeptide of active insulin as defined above, and which has at least approximately 80% identity of nucleic acid sequence with a nucleic acid sequence that codes for: (a) residues 1 to 21 of the A chain of the high-fat diet defined above in combination with residues 1 to 30 of the B chain of the human insulin polypeptide defined above, or (b) one of nucleic acid that codes for another fragment specifically derived from the aforementioned sequences of amino acids which shows at least 80%, 82%, 83%, 84%, 85%, 86%, 87%, 88%, 89%, 90%, 91% 92%, 93%, 94%, 95%, 96%, 97%, 98% or 99% of identity of amino acid sequence with said sequences. The polynucleotides which code for a variant of insulin contain a nucleic acid that codes for an A chain of insulin of at least approximately 45, 48, 51, 54, 57, 60, 63, 66, 69, 72, 75, 81, 87, 105 nucleotides and/or a nucleic acid that codes for a B chain of insulin of at least approximately 75, 78, 81, 84, 87, 90, 93, 96, 99, 102, 105, 108, 111, 114, 117, 120, 123, 126, 129, 132, 135 or 150 nucleotides.

Said polynucleotides may appear isolated or be found as part of a vector. Suitable vectors for the expression of the polynucleotides that code for insulin or the functionally equivalent variant thereof are essentially the same as those previously described in relation to the composition comprised by CT-1.

Alternatively, the anti-diabetic compounds that can be used in the context of the present invention include compounds which that antiglycaemic activity of the insulin or sensitizing agents of insulin activity and include, without limitation, secretagogues of insulin such as the sulfonylureas (tolbutamide, chlorpropamide, glipicide, glibenclamide, glicazide, glipentide, glimepiride, glibenclamide, glipizide, gliquidone, glisentide, glimepride and such like) and metiglinides (repaglinide, nateglinide, mitiglinide and such like), reducing agents of liver glucose production (biguanides and, in particular, metformin and buformin), agents which cause carbohydrate decrease such as α-glucosidase inhibitors (acarbose, miglitol or voglibose), agents that increase peripheral use of glucose such as thiazolidinediones (rosiglitazone, pioglitazone and such like), GLP- or a GLP-1 mimetic (Byetta-Exanatide, Liraglutinide, CJC-1131 (ConjuChem, Exanatide-LAR (Amylin), BIM-51077, ZP-10, the compounds described in WO 00/07617 and such like), exendin, secretin, DPP-IV inhibitors (sitagliptin, saxagliptin, denagliptin, vildagliptin, ALS-2-0426, ARI- 2243, BI-A, BI-B, SYR-322, MP-513, DP-893, RO-0730699 and such like), SGLT-2 inhibitors (dapagliflozin, and sergliflozin, AVE2268, T- 1095 and such like) and peptides that cause an increase in glucose production (amlintide, pramlintide, exendin), compounds with GLP-1 activity (glucagon-like peptide 1), inhibitors of the protein tyrosine phosphatase 1B, dipeptidyl peptidase inhibitors, secretagogues of insulin; fatty acid oxidation inhibitors, A2 antagonists, c-jun terminal kinase inhibitors, insulin; insulin mimetics, glycogen phsophorylase inhibitors, VPAC2 receptor agonists, glucokinase inhibitors, etc.).

The compositions of the invention can be used for the treatment of metabolic disease given their hypoglycaemic effects and their capacity to increase glucose capture by muscles (see example 2). Said compositions can be used as preparations of the different compounds for their administration simultaneously, separately or sequentially. Thus, in another aspect, the invention is related to a composition of the invention for the treatment of a metabolic disease wherein the composition is administered simultaneously, separately or sequentially. In another aspect, the invention is related to the use of a composition of the invention for the preparation of a drug for the treatment of a metabolic disease wherein the composition is administered simultaneously, separately or sequentially. In another aspect, the invention is related to a method for the treatment of a metabolic disease which comprises the simultaneous, separate or sequential administration of a composition of the invention.

The therapeutic agents of the compositions of the invention, in particular, the CT-1 activity inducer agent and the anti-diabetic agent, can be presented as a single formulation (for example, as a tablet or a capsule comprising a fixed quantity of each one of the components) or can, on the other hand, be presented as separate formulations to be later combined for joint, sequential, or separate administration. The compositions of the invention also include the formulation as a kit-of-parts wherein the components are formulated separately but are packaged in the same container. Those skilled in the art will appreciate that the formulation of the first and second components of the compositions of the invention may be similar, in other words, similarly formulated (in tablets or pills), which allows their administration by the same route. In the case where the different components of the invention are formulated separately, the two components can be presented in a blister. Each blister contains the drugs that must be consumed during the day. If the drugs must be administered several times a day, the drugs corresponding to each administration can be placed in different sections of the blister, preferably recording in each section of the blister the time of day when they should be administered. Alternatively, the components of the composition of the invention can be formulated differently so that the different components are differently administered. Thus, it is possible that the first component is formulated as a tablet or capsule for its oral administration and the second component is formulated for its intravenous administration.

The compositions of the invention are administered by the methods known to those skilled in the art, including, but without limitation, intravenous, oral, nasal, parenteral, topical, transdermal, rectal, and such like.

The ratio between the components that are part of the compositions of the invention shall depend on the CT-1 activity inducer agent and the anti-diabetic agent used in each particular case, as well as of the desired indication. Thus, the invention envisages compositions wherein the ratio between the quantities of the two components can range from 50:1 to 1:50, in particular from 20:1 to 1:20, from 1:10 to 10:1, or from 5:1 to 1:5.

The metabolic diseases that can be treated with the compositions of the invention include, without limitation, obesity, insulin resistance, hyperglycaemia, dyslipemia, and type 2 diabetes, as has been previously described in relation to the first aspect of the invention (therapeutic and cosmetic uses of CT-1). In another embodiment, the invention is related to a cosmetic method for the treatment of obesity which comprises administering a patient a composition in accordance with the invention, wherein those patients candidate to an anti-obesity treatment for purely cosmetic reasons are identified as has been described in the first aspect of the invention (therapeutic and cosmetic uses of CT-1).

The invention is described below by the following examples that should be considered merely illustrative and in no case limitative of the scope of the present invention.

### EXAMPLES

### EXAMPLE 1

### Effect of CT-1 on body weight

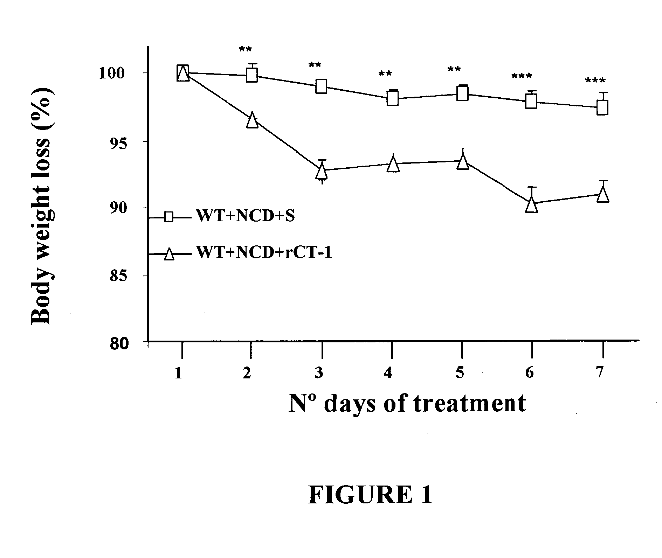

Obese mice were obtained by the intake of a high-fat diet (HFD, 60% fat) during 3 months. The intravenous administration for 6 consecutive days of rCT-1 (0.2mg/kg/day) caused a reduction in the body weight of C57BL/6 mice (5 months of age) with high-fat diet (HFD) (Fig. 1). The dose used in this experiment did not produce a feverish reaction (rectal temperature was measured during all days of treatment).

The decrease in body weight observed in the obese mice, (fed HFD as described in figure 1) and treated with rCT-1 for 6 consecutive days at the aforementioned dose 0.2mg/kg/day, is due to the anorexigenic effect of this cytokine (fig. 2). The figure shows the kilocalories ingested during the 6 days of treatment of the group treated with rCT-1 and its corresponding control group treated with physiological serum intravenously (S).

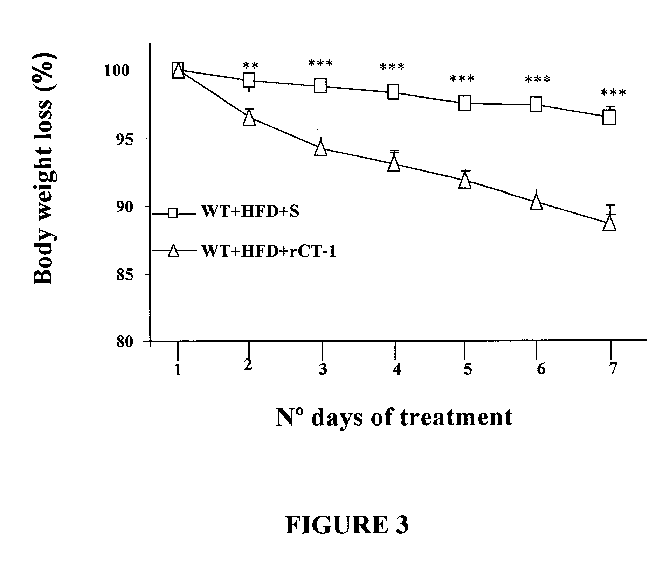

Likewise, the intravenous administration of rCT-1 (0.2mg/kg/day) for 6 consecutive days reduced body weight in non-obese C57BL/6 mice (5 months of age) fed a normal diet (NCD) (Fig. 3). The dose used in this experiment did not produce feverish reaction (the rectal temperature was measured during all days of treatment).

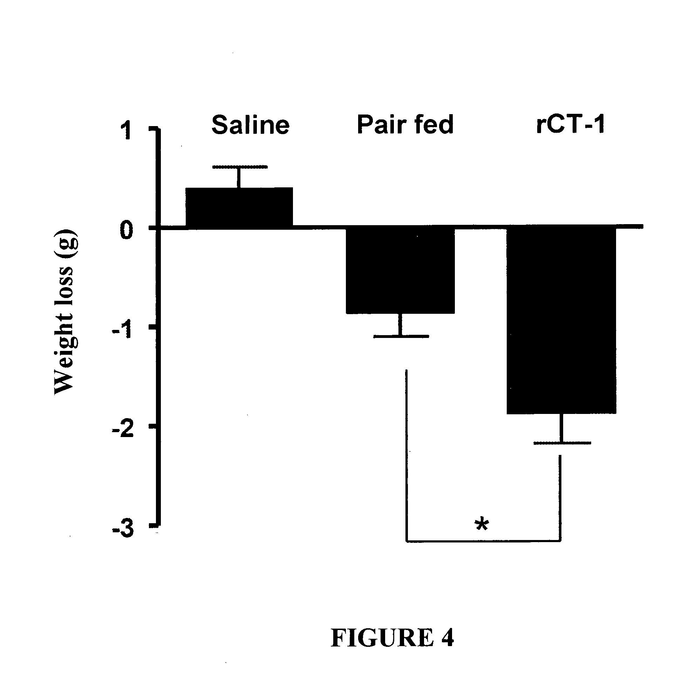

With the purpose of determining whether the reduction in body weight observed during treatment with rCT-1 with the dose and in the previously commented form was due to an anorexigenic effect or involved other effects, a study was performed using 3 groups of C57BL/6 mice of 4 months of age. The first group (Saline group) received a physiological serum intravenously (the same volume as that used for the administration of rCT-1); the second group (rCT-1 group) received rCT-1 intravenously at the aforementioned dose (0.2mg/kg/day) and the third group (Pair-Fed group) received the same quantity of food daily as that ingested by the mice treated with rCT-1 and which were injected with serum during the same experimental period. As observed in figure 4, the mice treated with rCT-1 lost more weight than the pair-fed group, despite the energy intake being identical in both groups, indicating that in addition to the previously observed anorexigenic effect, rCT-1 would have other metabolic effects responsible for weight loss.

### EXAMPLE 2

### Hypoglycaemic effects of CT-1

With the purpose of determining whether the acute intravenous administration of a single dose of rCT-1 (10µg) to C57BL/6 mice (3 months of age) reduced the baseline blood sugar levels, the blood glucose levels were measured 1 hour after treatment with rCT-1 and saline serum. As observed in figure 5A, rCT-1 caused a significant decrease in blood sugar levels. The mechanism whereby the blood sugar decreased cannot be attributed to an increase in blood insulin levels (as shown in figure 5B), since no appreciable differences are observed in the insulin levels of the animals treated with CT-1 or with saline serum. The results are expressed as mean ± SE. n=5 animals per group.*p<0.05. Treatment with rCT-1 induces phosphorylation of AKT in muscle, which can contribute to explaining the hypoglycaemic action of this cytokine (figure 5C shows a representative Western).

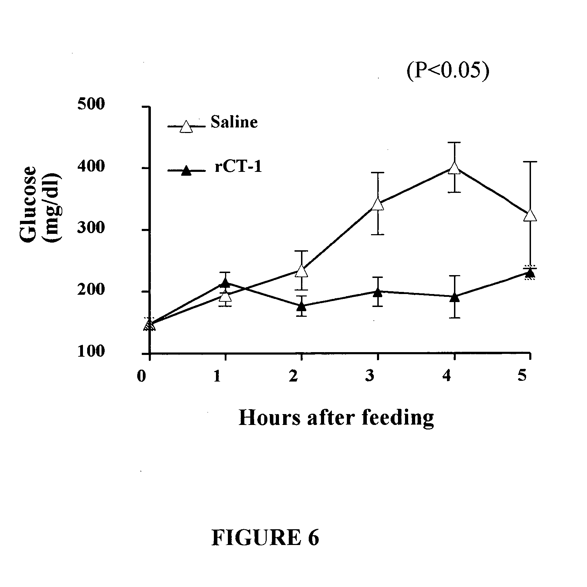

The acute administration of a single dose of rCT-1 (10µg) prevents the increase of blood glucose after a high sugar diet. This experiment to study the effects of rCT-1 on the post-pandrial blood sugar was performed on mice (C57BL/6, 3 months of age) administered a high sugar diet by gastric gavage (in a volume 1% body weight) in accordance with that published by Fruebis et al. (Fruebis et al., 2001, Proc.Natl.Acad.Sci.USA., 98:2005-2010). After performing the gavage, the intravenous administration of the same volume of saline serum was given (Saline) or rCT-1 (10µg). Blood was taken at the hours indicated in the graphic and blood sugar was measured observing that no increase in blood sugar occurred in the animals treated with rCT-1 (Fig. 6).

Next, it was determined whether acute administration of a single rCT-1 dose decreases blood sugar levels in mice (C57BL/6, 3 months of age) was capable of decreasing hyperglycaemia secondary to the destruction of beta-pancreatic cells performed with treatment with streptozotocin. Therefore, 64 hours after administration of a single dose of streptozotocin (200mg/kg), the baseline blood sugar was measured and the different treatments were performed on 3 groups of mice. The first group (saline group) was treated with treated with saline serum; the second group (Insulin group) was treated with 0.75U/kg insulin, the third group (rCT-1 group) was treated with 10µg of rCT-1 and the fourth group (Insulin group + rCT-1) was treated with 0.75U/kg of insulin and 10µg of rCT-1. Once the different treatments have been administered, blood sugar was measured after 15, 30, 60 and 120 min. As observed in figure 7, rCT-1 significantly decreased blood sugar 30 min after its administration and enhanced the effects of insulin in all blood extraction points analysed.

In order to determine if the hypoglycaemic effect resulting from acute treatment with rCT-1 is due to an increase in glucose capture by muscles, muscular capture of 18FDG (18 FluoroDeoxyGlucose) was determined by gamma counter in different groups of mice. To perform this experiment, the mice (C57BL/6, 3 months of age) were divided into 4 groups as follows: Saline group (treated with saline serum); Insulin group (treated with 0.75U/kg insulin), rCT-1 group (treated with 10µg of rCT-1) and Insulin group + rCT-1 (treated with 0.75U/kg of insulin + 10µg of rCT-1. 15 minutes after the administration of the different treatments 18FDG was administered and 10 minutes after the injection the gastrocnemius muscles were extracted, and the radioactivity emitted was quantified by gamma counter. The results (Fig. 8) show that rCT-1 may increase glucose consumption by muscles and enhance (although not significantly with respect to those observed with insulin).

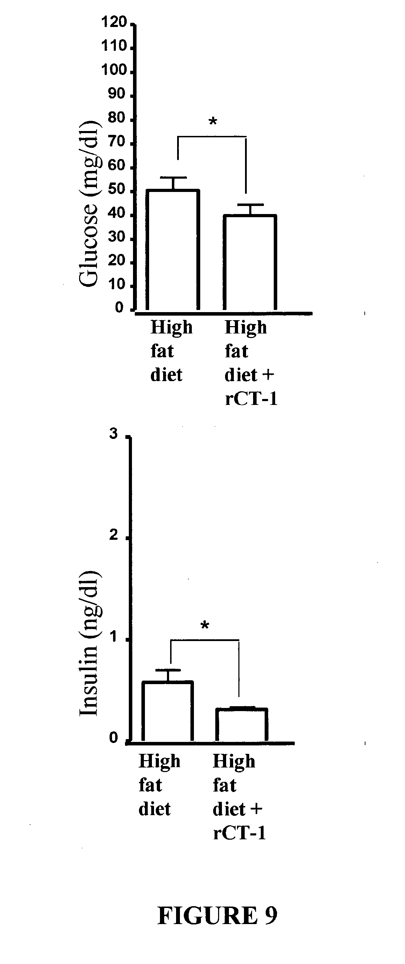

Next, the hypoglycaemic and hypoinsulinemic effects of chronic treatment with rCT-1 were determined in C57BL/6 mice (5 months of age) with obesity induced by the intake of a high-fat diet (HFD, 60% fat) for 3 months. At the end of the dietary treatment, the mice were treated for 6 consecutive days (chronic treatment) with rCT-1 (0.2mg/kg/day) administered endovenously. As shown in figure 9, the blood sugar levels (upper panel) and insulinemia levels (lower panel) decreased significantly due to treatment with rCT-1.

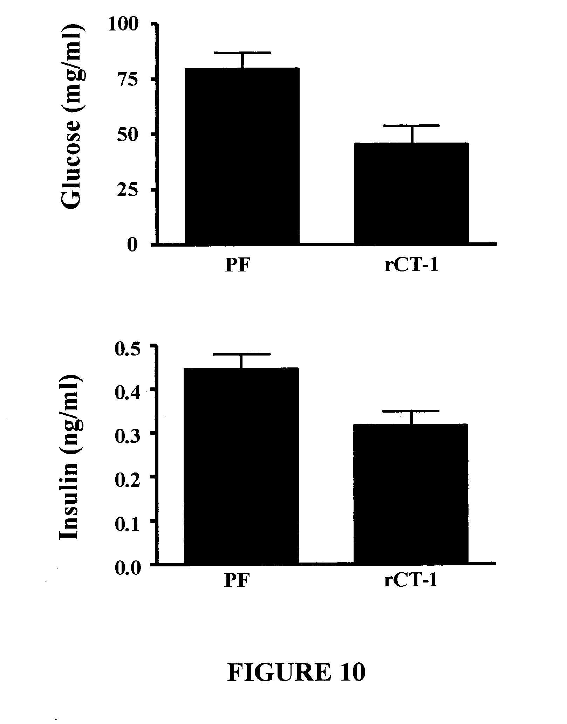

Next, the hypoglycaemic and hypoinsulinemic effects of chronic treatment with rCT-1 were compared with their corresponding pair-fed (PF) group. The rCT-1 group received rCT-1 by intravenous route at the aforementioned dose (0.2mg/kg/day) for 6 days; the Pair-Fed group received the same quantity of daily food as that ingested by the mice treated with rCT-1 and serum was injected into them for the same experimental period. As shown in figure 10, a greater drop in blood sugar (upper panel) was demonstrated in animals subjected to chronic treatment with rCT-1 than in those subjected to a similar calorie restriction, suggesting that the hypoglycaemic effects are not only due to the anorexigenic effect induced by rCT-1, but they seem due to the hypoglycaemic properties of the protein *per se.*

Then, it was determined whether the *in vitro* treatment with rCT-1 (0-100 ng/ml) is capable of inhibiting the transport of sugar in intestinal rings. Therefore, the intestinal transport of glucose was determined by measuring the capture of α-methylglucoside (1 mM) by everted jejunum rings for 15 minutes of incubation in the absence or presence of r-CT-1 at 37° C. The α-methylglucoside is an analog of the glucose which only crosses the apical membrane of the enterocyte by the SGLT1 sodium-sugar co-transcarrier, which means that the changes in their transport should be attributed to changes in the activity of said transcarrier. Figure 11 shows that CT-1 is capable of inhibiting the absorption of glucose in the intestine although the effect observed is not dose-dependent.

Next, the actions of CT-1 and CNTF on the capture of α-methylglucoside (1 mM) in intestinal rings was determined. Following the experimental method described in figure 11, it was observed that the inhibitory action of CT-1 on the intestinal transport of glucose is greater than that of CNTF (which did not achieve significant effects in the experimental conditions tested).

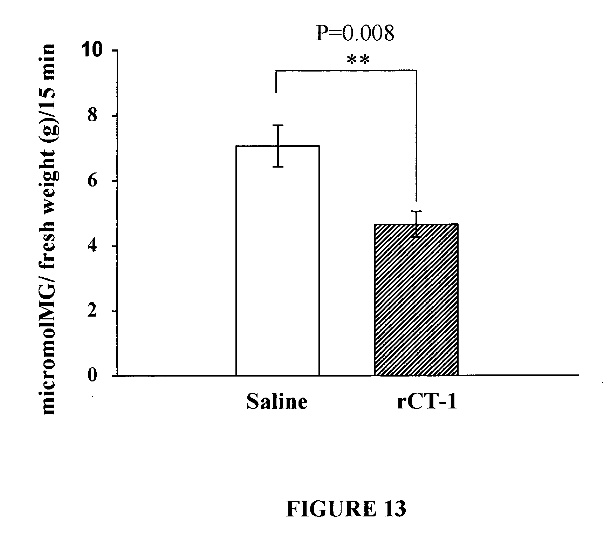

Next, it was determined whether the in vitro effects of CT-1 on the intestinal capture of glucose could be reproduced in vivo. To do this, rCT-1 (10µg) was administered intravenously and acutely to C57BL/6 mice (2 months of age). It was observed that the administration of CT-1 caused an inhibition in the intestinal transport of α-methylglucoside (1 mM) (Fig. 13). The isolation of the intestinal rings and the transport of α-methylglucoside (1 mM) by the same was performed 3 hours after administration of CT-1. The results obtained indicate the capacity of CT-1 to inhibit the intestinal transport of glucose when it is administered systemically in the animal and reinforces the previous experiments performed *in vitro* which showed the capacity of rCT-1 to inhibit the transport of intestinal glucose.

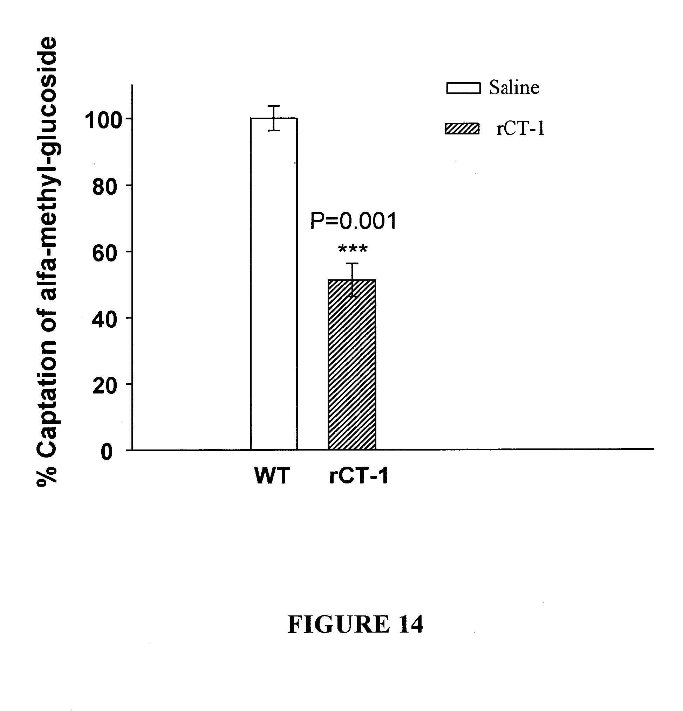

It was later determined whether the intravenous administration of rCT-1 (0.2 mg/kg/day) for 6 consecutive days (chronic treatment) to C57BL/6 mice (5 months of age) was capable of inhibiting the intestinal capture of α-methylglucoside (1 mM) in intestinal rings in comparison with those from mice treated with saline serum. The results are shown in figure 14, wherein it can be observed how rCT-1 is capable of inhibiting intestinal glucose transport (chronic treatment *in vivo*).

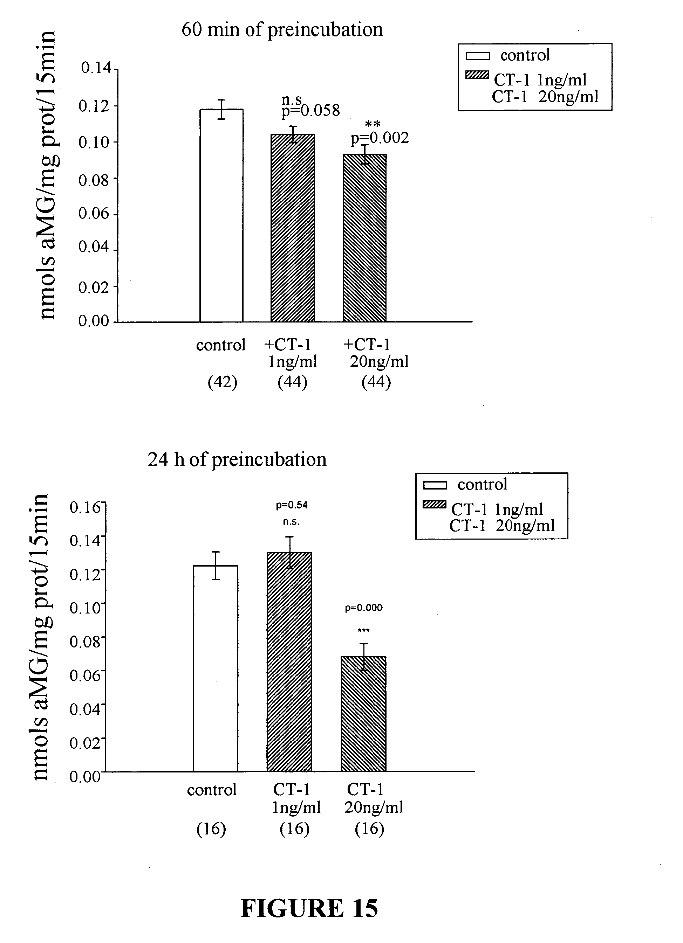

Next, the effect of rCT-1 was determined on the capture of α-methylglucoside (0.1 mM) by cells of the CACO-2 human cell line. The results (figure 15) show that treatment with rCT-1 causes a concentration-dependent inhibition of the capture of α-methylglucoside (0.1 mM) in the CACO-2 human cell line, which behaves functionally as human enterocytes. Treatment with CT-1 was performed from the apical edge for 1h (Fig. 15A) and 24h (Fig. 15B), observing significant differences with the dose of 20ng/ml in the two pre-incubation times studied. The results are expressed as mean ± SE of 42-44 determinations per group.

### EXAMPLE 3

### Differential effects of CT-1 with CNTF

Figure 16 shows the results of an experiment carried out on primary cultures of rat adipocytes to determine the capacity of rCT-1 and CNTF to modulate leptin secretion by adipocytes. Figure 16A shows how treatment with rCT-1 (72 h) inhibits both baseline secretion of leptin and that stimulated by insulin (1.6 nM). However, these effects were not observed with similar concentrations of CNTF (figure 16B).

Figure 17 shows the results of experiments made in primary cultures of rat adipocytes to determine the ability of CT-1 and CNTF to modulate lipolysis in the absence or presence of insulin. The figure demonstrates that CT-1 is able to induce the release of glycerol (lipolysis measurement) and also inhibits the anti-lipolytic activity of insulin, however CNTF is not able. These data indicate that CT-1 is able to mobilize fat that in addition, to the increased beta-oxidation in muscle shown previously, indicate the ability of this cytokine to reduce fatty deposits.

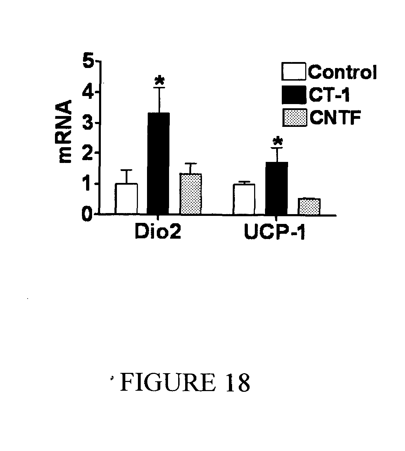

Aditionally and importantly, CT-1 is able to induce typical genes of brown adipose tissue in adipocytes (deiodinase iodothyronine type II, Dio-2 and uncoupling protein-1, UCP-1) while CNTF is not able (figure 18). Several studies have shown that therapies aimed at inducing genes typical of brown adipose tissue in white adipose tissue would be effective for the amelioration of obesity-related disorders (Farmer S. Genes Dev. 2008; 22:1269-7).

### EXAMPLE 4

### Chronic administration of CT-1 does not result in inflammation

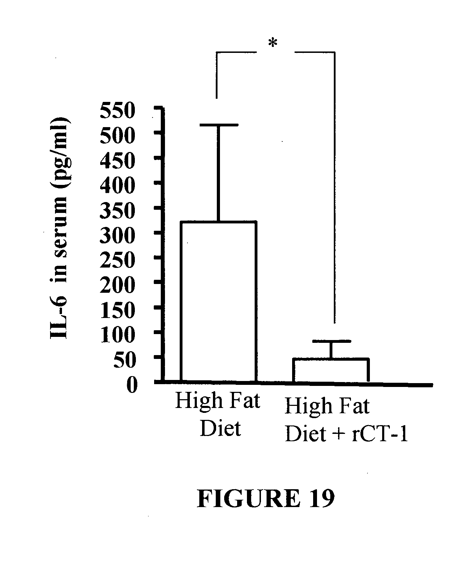

In order to determine the capacity of CT-1 of producing inflammation after its chronic administration, serum levels of IL-6 in C57BL/6 mice were determined (5 months of age) with obesity induced by the intake of a high-fat diet (HFD, 60% fat) for 3 months. At the end of the dietetic treatment, the mice were treated for 6 consecutive days (chronic treatment) with rCT-1 (0.2 mg/kg/day) administered endovenously. The results (figure 19) indicate that chronic treatment at the doses used did not cause inflammatory signs (data histologically confirmed in organs such as liver and muscle).

Additionally, histological cuts of liver were performed in order to detect by microscopy the appearance of symptoms of inflammation. The results (figure 20) show images representative of histological cuts of liver (H&E 100X) in 3 groups of mice (C57BL/6, 4 months of age): Control group: which received physiological serum by intravenous route (the same volume as that used for the administration of rCT-1); rCT-1 group: which received rCT-1 by intravenous route at the aforementioned dose (0.2mg/kg/day) for 6 consecutive days; Pair-Fed group: which received the same quantity of daily food as that the mice treated with rCT-1 would ingest and into which serum was injected during the same experimental period. As observed in the figure, in none of the three groups do we observe the presence of inflammatory infiltrate, nor any other significant morphological alteration.

### EXAMPLE 5

### Effect of CT-1 on lipid metabolism

With the purpose of determining the possible role of CT-1 on the lipid metabolism, the levels of different serum lipids were determined in mice with high-fat diet-induced obesity for 3 months after chronic administration with rCT-1 for 6 days. The results are shown in Table 1

**Table 1: Chronic treatment with rCT-1 for 6 consecutive days in mice with high-fat diet-induced obesity (HFD) for 3 months. * P < 0.05, ***P < 0.001**

| | **Obese mice with HFD** | |
|---|---|---|
| | **Vehicle (n=7)** | **CT-1 (n=7)** |
| **Total Cholesterol (mg/dl)** | 314.8 ± 22.30 | 186.6±7.8*** |
| **LDL-Cholesterol(mg/dl)** | 193.4 ± 21.6 | 937 ± 47*** |
| **Total Cholesterol/** | 3.22 ± 0.35 | 2.41 ± 0.06* |
| **HDL-cholesterol ratio Triglycerides (mg/dl)** | 99.9 ± 10.26 | 76.8 ± 7.63* |
| **Free Fatty Acids (mmol/l)** | 1.87 ± 0.11 | 0.800.03 |
| **Glucose (mg/dl)** | 212.4 ± 26.54 | 138.6 ± 17.27* |
| **Insulin (ng/ml)** | 2.08 ± 0.76 | 1.01 ± 0.24* |

Additionally, the serum levels of fatty acids and triglycerides were determined in wild mice after a high-fat diet. To do this, two groups of animals were used (n=5) which remained fasting for 3 hours before the experiment. Next, both groups of animals were administered high fat food by gavage (1% of body weight) and a blood sample was obtained to determine the baseline levels. Immediately after the food, serum (▲) or rCT-1 (10 µg/ml) (Δ) was administered by intravenous route. The results (figure 21) show how treatment with rCT-1 leads to a significant reduction in the free fatty acid levels (after 2-5 hours, **P*<0.05) (upper panel) and triglyceride levels (after 1-3 hours, **P*<0.05) (lower panel).

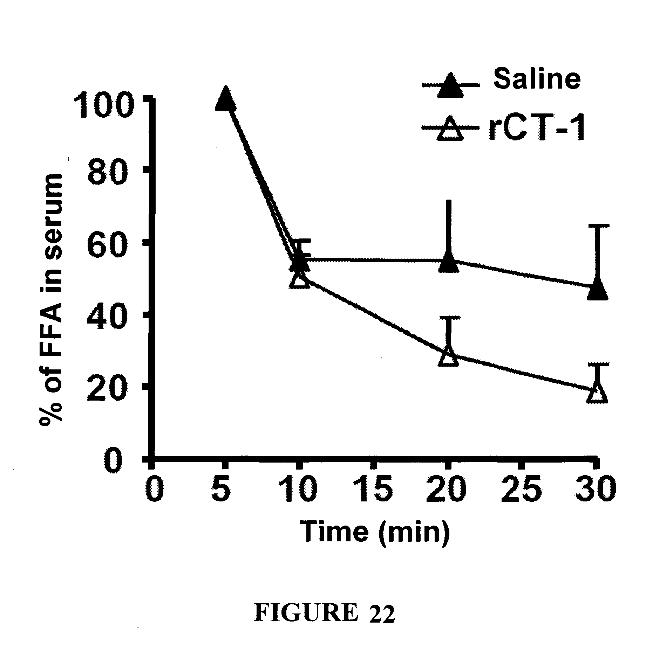

Then, it was investigated if treatment with rCT-1 accelerated the elimination of free fatty acids in serum after an intralipid injection. To do this, rCT-1 was administered intravenously to two groups of animals (n=5) 30 minutes after administration of intralipids. The control animals received saline. The concentration of free fatty acids in serum was determined after 5 minutes. Then, the elimination values of the free fatty acids taken at different times were normalized with respect to the FFA values 5 minutes after the injection of intralipids (100%). As shown in figure 22, the abulia kinetics of the FFA significantly differered between the mice treated with saline and with CT-1 (*P*<0.05 by two-way ANOVA with Bonferroni analysis *post hoc*).

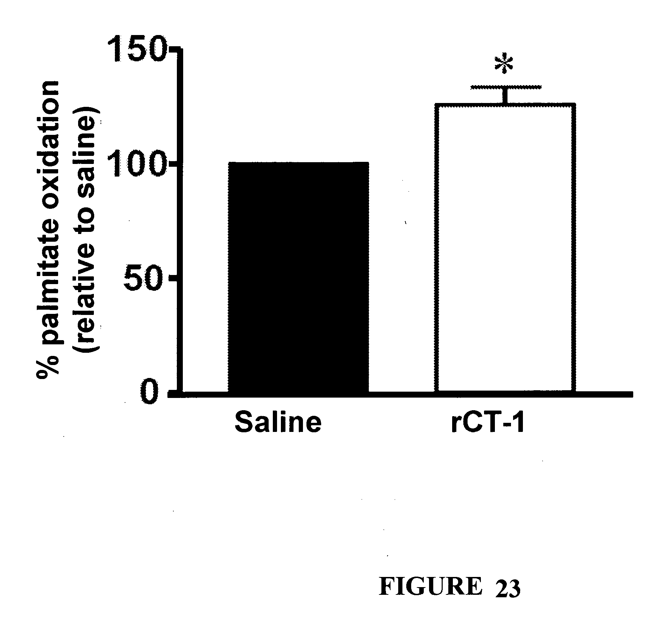

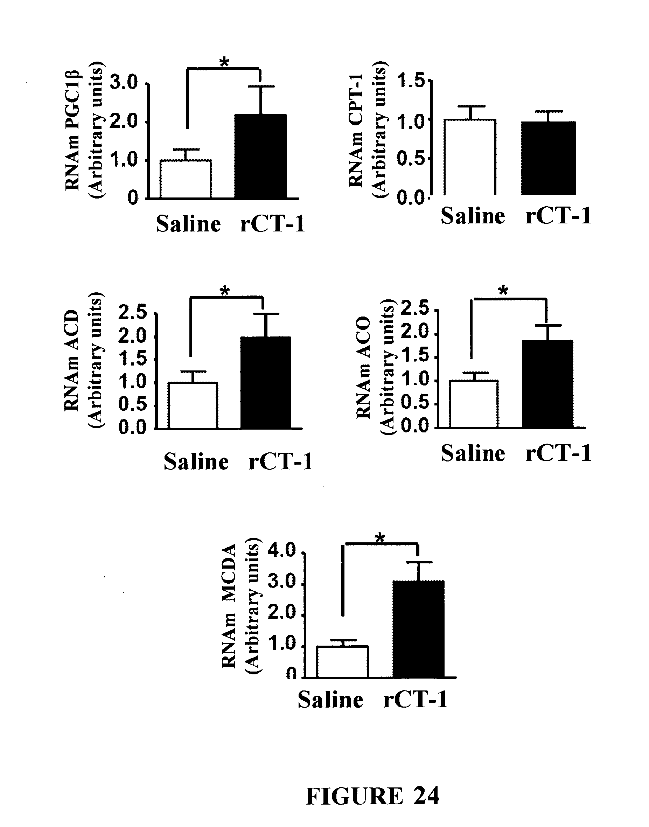

In order to determine the possible effects of rCT-1 on beta oxidation, the oxidation of palmiate in gastrocnemius muscle isolated from mice treated with saline or with CT-1 (10 µg for 1h) was determined. The results (figure 23) show that acute treatment with rCT-1 causes an increase of the oxidation of fatty acids in the skeletal muscle. Additionally, the expression levels of genes involved in the oxidation of fatty acids in muscles were determined by RT-PCR in response to acute treatment with rCT-1 (3 hours). The results (figure 24) show that rCT-1 causes an increase in mRNA levels of the PGC-1b, CPT-1, ACD, ACO and MCDA genes. The number of cycles necessary to detect each one of the transcripts was compared with the number corresponding to cyclophylin as internal control and it is expressed as arbitrary units compared with values in the animals treated with saline taken as 1.0 (n=5-6, **P*<0.05).

### EXAMPLE 6

### Enhancing effect of CT-1 on insulin action in skeletal muscle

Skeletal muscle is the most sensitive tissue to insulin action and provides in healthy individuals for 90% of glucose uptake stimulated by insulin. The authors have shown that CT-1 enhances insulin signaling in muscle "*in vitro*" and *"in vivo*".

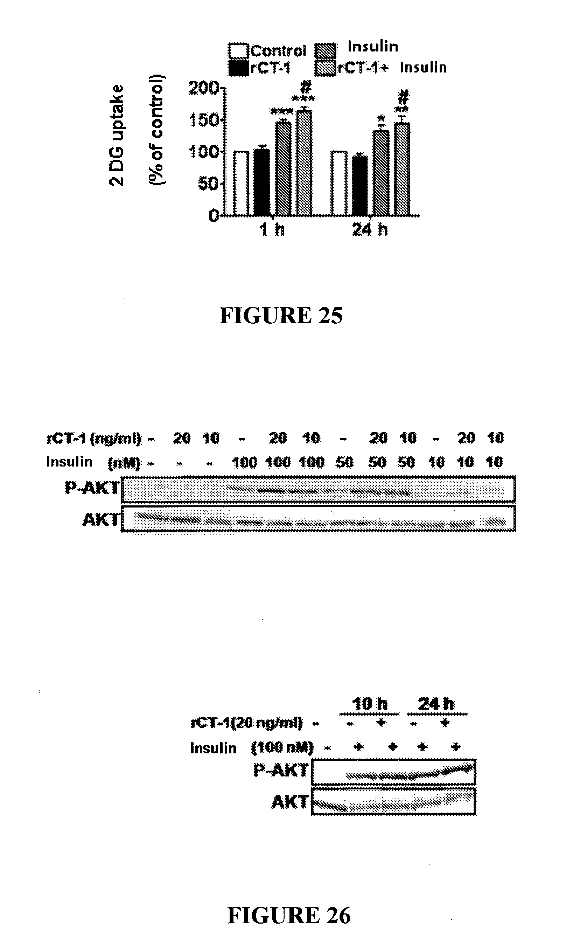

The experiments "*in vitro*" were carried by measuring insulin-induced 2-deoxyglucose uptake and AKT phosphorylation in rat myotubes (L6E9 myoblasts differentiated to myotubes) after acute (1 hour) or chronic treatment (24 hours) with rCT-1. It was observed that CT-1 stimulates insulin-induced 2-deoxyglucose uptake (figure 25) and insulin-activated AKT phosphorylation (figure 26). These data demonstrate that CT-1 enhances the action of insulin in muscle. These data differ from those published for the cytokine from the same family, IL-6 (Nieto-Vazquez, I. et al., Diabetes 2008, 57: 3211-3221).

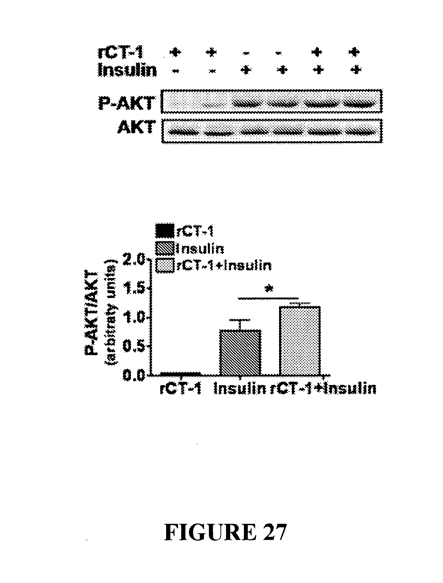

Moreover, it was observed "*in vivo*" that intravenous administration of rCT-1 (10 µg / mouse) 30 minutes before insulin stimulation (0.5 U / mouse injected into the inferior cave vein) increased insulin-induced AKT phosphorylation in muscle (figure 27). Furthermore, chronic treatment with rCT-1 during 6 consecutive days at doses of 0.2 mg / kg / day intravenously enhanced the effects of insulin in muscle tissue compared with animals with the same intake than those treated with CT-1 and being injected with saline serum (pair-fed group) (figure 28). All these data demonstrate the stimulatory role of CT-1 in the presence of insulin in skeletal muscle.

## Claims

1. A compound that induces cardiotrophin 1 (CT-1) activity selected from the group consisting of
(i) Cardiotrophin-1 (CT-1),
(ii) a functionally equivalent variant of CT-1 showing an identity of sequence with human CT-1 of at least 90 %,
(iii) a polynucleotide that codes for CT-1 or a functionally equivalent variant thereof showing an identity of sequence with human CT-1 of at least 90 %,
(iv) a vector that comprises a polynucleotide according to (iii) and
(v) a cell capable of secreting into the medium cardiotrophin-1 or a functionally equivalent variant thereof showing an identity of sequence with human CT-1 of at least 90 %
for use in the treatment of a metabolic disease, wherein the metabolic disease is selected from the set of obesity, hyperglycaemias, insulin resistance, type 2 diabetes, and dyslipidemia, wherein the identity of sequence is determined using BLASTP algorithm.

2. Use of a compound that induces cardiotrophin 1 (CT-1) activity selected from the group consisting of
(i) Cardiotrophin-1 (CT-1),
(ii) a functionally equivalent variant of CT-1 showing an identity of sequence with human CT-1 of at least 90 %,
(iii) a polynucleotide that codes for CT-1 or a functionally equivalent variant thereof showing an identity of sequence with human CT-1 of at least 90 %,
(iv) a vector that comprises a polynucleotide according to (iii) and
(v) a cell capable of secreting into the medium cardiotrophin-1 or a functionally equivalent variant thereof showing an identity of sequence with human CT-1 of at least 90 %
for the preparation of a drug for the treatment of a metabolic disease, wherein the metabolic disease is selected from the set of obesity, hyperglycaemia, insulin resistance, type 2 diabetes, and dyslipidemia, wherein the identity of sequence is determined using BLASTP algorithm.

3. Cosmetic method for the treatment of grade 1 overweight (25.0-29.9 kg/m²), which comprises administering a patient a pharmaceutically active quantity of a compound that induces cardiotrophin 1 activity selected from the group of
(i) Cardiotrophin-1 (CT-1),
(ii) a functionally equivalent variant of CT-1 showing an identity of sequence with human CT-1 of at least 90 %,
(iii) a polynucleotide that codes for CT-1 or a functionally equivalent variant thereof showing an identity of sequence with human CT-1 of at least 90 %,
(iv) a vector that comprises a polynucleotide according to (iii) and
(v) a cell capable of secreting into the medium cardiotrophin-1 or a functionally equivalent variant thereof showing an identity of sequence with human CT-1 of at least 90 %
wherein the identity of sequence is determined using BLASTP algorithm.

4. A compound for use according to claim 1, a use according to claim 2 or a method according to claim 3, wherein said compound is administered for acute or chronic treatment.

5. A composition that comprises, together or separately, a compound that induces cardiotrophin activity selected from the group consisting of
(i) Cardiotrophin-1,
(ii) a functionally equivalent variant of CT-1 showing an identity of sequence with human CT-1 of at least 90 %,
(iii) a polynucleotide that codes for CT-1 or a functionally equivalent variant thereof showing an identity of sequence with human CT-1 of at least 90 %
(iv) a vector that comprises a polynucleotide according to (iii) and
(v) a cell capable of secreting into the medium cardiotrophin-1 or a functionally equivalent variant thereof showing an identity of sequence with human CT-1 of at least 90 %
and an anti-diabetic compound, wherein the identity of sequence is determined using BLASTP algorithm.

6. A composition according to claim 5, wherein the anti-diabetic compound is selected from the set of a compound that induces insulin activity and a compound that induces the hypoglycaemic activity and/or sensitizing agent to insulin activity.

7. A composition according to claim 6, wherein the compound that induces insulin activity is selected from the set of:
(i) insulin,
(ii) a polynucleotide that codes for insulin and
(iii) a vector that comprises a polynucleotide according to (ii)

8. A composition according to claims 5 or 7 for use in the treatment of a metabolic disease, wherein the composition is administered simultaneously, separately or sequentially and wherein the metabolic disease is selected from the set of obesity, insulin resistance, hyperglycaemia, dyslipidemia, and type 2 diabetes.

9. Use of a composition according to claims 5 or 7 for the preparation of a drug for the treatment of a metabolic disease, wherein the composition is administered simultaneously, separately, or sequentially.

10. Cosmetic method for the treatment of grade 1 overweight (25.0-29.9 kg/m²) which comprises administering the patient a pharmaceutically active quantity of a composition according to of claims 5 to 7.

11. Method according to claim 10, wherein the treatment is acute or chronic.

## Patentansprüche

1. Verbindung, die Cardiotrophin-1 (CT-1) Aktivität induziert, die ausgewählt ist aus der Gruppe bestehend aus
(i) Cardiotrophin-1 (CT-1),
(ii) eine funktionell äquivalente Variante von CT-1, die eine Sequenzidentität mit menschlichem CT-1 von mindestens 90% aufweist,
(iii) ein Polynucleotid, das CT-1 oder eine funktionell äquivalente Variante davon, die eine Sequenzidentität mit menschlichem CT-1 von mindestens 90% aufweist, codiert,
(iv) ein Vektor, der ein Polynucleotid nach (iii) umfasst, und
(v) eine Zelle, die dazu fähig ist, Cardiotrophin-1 oder eine funktionell äquivalente Variante davon, die eine Sequenzidentität mit menschlichem CT-1 von mindestens 90% aufweist, in das Medium zu sekretieren,
zur Verwendung bei der Behandlung von einer Stoffwechselerkrankung, wobei die Stoffwechselerkrankung ausgewählt ist aus der Gruppe von Fettleibigkeit, Hyperglykämie, Insulinresistenz, Typ-2-Diabetes und Dyslipidämie, wobei die Sequenzidentität durch Verwendung von BLASTP-Algorithmus bestimmt wird.

2. Verwendung einer Verbindung, die Cardiotrophin-1 (CT-1) Aktivität induziert, die ausgewählt ist aus der Gruppe bestehend aus
(i) Cardiotrophin-1 (CT-1),
(ii) eine funktionell äquivalente Variante von CT-1, die eine Sequenzidentität mit menschlichem CT-1 von mindestens 90% aufweist,
(iii) ein Polynucleotid, das CT-1 oder eine funktionell äquivalente Variante davon, die eine Sequenzidentität mit menschlichem CT-1 von mindestens 90% aufweist, codiert,
(iv) ein Vektor, der ein Polynucleotid nach (iii) umfasst, und
(v) eine Zelle, die dazu fähig ist, Cardiotrophin-1 oder eine funktionell äquivalente Variante davon, die eine Sequenzidentität mit menschlichem CT-1 von mindestens 90% aufweist, in das Medium zu sekretieren,
zur Herstellung eines Medikaments zur Behandlung einer Stoffwechselerkrankung, wobei die Stoffwechselerkrankung ausgewählt ist aus der Gruppe von Fettleibigkeit, Hyperglykämie, Insulinresistenz, Typ-2-Diabetes und Dyslipidämie, wobei die Sequenzidentität durch Verwendung von BLASTP-Algorithmus bestimmt wird.

3. Kosmetisches Verfahren zur Behandlung von Grad 1 Übergewicht (25.0 - 29.9 kg/m²), umfassend das Verabreichen einer pharmazeutisch wirksamen Menge einer Verbindung an einen Patienten, die Cardiotrophin-1 (CT-1) Aktivität induziert, die ausgewählt ist aus der Gruppe von
(i) Cardiotrophin-1 (CT-1),
(ii) eine funktionell äquivalente Variante von CT-1, die eine Sequenzidentität mit menschlichem CT-1 von mindestens 90% aufweist,
(iii) ein Polynucleotid, das CT-1 oder eine funktionell äquivalente Variante davon, die eine Sequenzidentität mit menschlichem CT-1 von mindestens 90% aufweist, codiert,
(iv) ein Vektor, der ein Polynucleotid nach (iii) umfasst, und
(v) eine Zelle, die dazu fähig ist, Cardiotrophin-1 oder eine funktionell äquivalente Variante davon, die eine Sequenzidentität mit menschlichem CT-1 von mindestens 90% aufweist, in das Medium zu sekretieren,
wobei die Sequenzidentität durch Verwendung von BLASTP-Algorithmus bestimmt wird.

4. Verbindung zur Verwendung nach Anspruch 1, Verwendung nach Anspruch 2 oder Verfahren nach Anspruch 3, wobei die Verbindung zur akuten oder chronischen Behandlung verabreicht wird.

5. Zusammensetzung, die zusammen oder getrennt eine Verbindung umfasst, die Cardiotrophin-1 (CT-1) Aktivität induziert, die ausgewählt ist aus der Gruppe bestehend aus
(i) Cardiotrophin-1 (CT-1),
(ii) eine funktionell äquivalente Variante von CT-1, die eine Sequenzidentität mit menschlichem CT-1 von mindestens 90% aufweist,
(iii) ein Polynucleotid, das CT-1 oder eine funktionell äquivalente Variante davon, die eine Sequenzidentität mit menschlichem CT-1 von mindestens 90% aufweist, codiert,
(iv) ein Vektor, der ein Polynucleotid nach (iii) umfasst, und
(v) eine Zelle, die dazu fähig ist, Cardiotrophin-1 oder eine funktionell äquivalente Variante davon, die eine Sequenzidentität mit menschlichem CT-1 von mindestens 90% aufweist, in das Medium zu sekretieren,
und eine anti-diabetische Verbindung, wobei die Sequenzidentität durch Verwendung von BLASTP-Algorithmus bestimmt wird.

6. Zusammensetzung nach Anspruch 5, wobei die anti-diabetische Verbindung ausgewählt ist aus der Gruppe von einer Verbindung, die Insulinaktivität induziert und einer Verbindung, die hyperglykämische Aktivität induziert und/oder Sensibilisator für Insulinaktivität.

7. Zusammensetzung nach Anspruch 6, wobei die Verbindung, die Insulinaktivität induziert, ausgewählt ist aus der Gruppe von:
(i) Insulin,
(ii) ein Polynucleotid, das Insulin codiert, und
(iii) ein Vektor, der ein Polynucleotid nach (ii) umfasst.

8. Zusammensetzung nach Anspruch 5 oder 7 zur Verwendung bei der Behandlung einer Stoffwechselerkrankung, wobei die Zusammensetzung gleichzeitig, getrennt oder sequentiell verabreicht wird und wobei die Stoffwechselerkrankung ausgewählt ist aus der Gruppe von Fettleibigkeit, Insulinresistenz, Hyperglykämie, Dyslipidämie und Typ-2-Diabetes.

9. Verwendung der Zusammensetzung nach Anspruch 5 oder 7 zur Herstellung eines Medikaments zur Behandlung einer Stoffwechselerkrankung, wobei die Zusammensetzung gleichzeitig, getrennt oder sequentiell verabreicht wird.

10. Kosmetisches Verfahren zur Behandlung von Grad 1 Übergewicht (25.0 - 29.9 kg/m²), umfassend das Verabreichen einer pharmazeutisch wirksamen Menge einer Zusammensetzung nach einem der Ansprüche 5 bis 7 an einen Patienten.

11. Verfahren nach Anspruch 10, wobei die Behandlung akut oder chronisch ist.

## Revendications

1. Composé qui induit une activité de cardiotrophine-1 (CT-1) sélectionné dans le groupe consistant en
(i) la cardiotrophine-1 (CT-1),
(ii) un variant fonctionnellement équivalent de la CT-1 montrant une identité de séquence avec la CT-1 humaine d'au moins 90 %,
(iii) un polynucléotide qui code pour la CT-1 ou un variant fonctionnellement équivalent de celle-ci montrant une identité de séquence avec la CT-1 humaine d'au moins 90 %,
(iv) un vecteur qui comprend un polynucléotide selon (iii) et
(v) une cellule capable de sécréter dans le milieu la cardiotrophine-1 ou un variant fonctionnellement équivalent de celle-ci montrant une identité de séquence avec la CT-1 humaine d'au moins 90 % pour son utilisation dans le traitement d'une maladie métabolique, où la maladie métabolique est sélectionnée dans le groupe consistant en l'obésité, l'hyperglycémie, l'insulinorésistance, le diabète de type 2, et la dyslipidémie, où l'identité de séquence est déterminée en utilisant l'algorithme BLASTP.

2. Utilisation d'un composé qui induit une activité de cardiotrophine-1 (CT-1) sélectionné dans le groupe consistant en
(i) la cardiotrophine-1 (CT-1),
(ii) un variant fonctionnellement équivalent de la CT-1 montrant une identité de séquence avec la CT-1 humaine d'au moins 90 %,
(iii) un polynucléotide qui code pour la CT-1 ou un variant fonctionnellement équivalent de celle-ci montrant une identité de séquence avec la CT-1 humaine d'au moins 90 %,
(iv) un vecteur qui comprend un polynucléotide selon (iii) et
(v) une cellule capable de sécréter dans le milieu la cardiotrophine-1 ou un variant fonctionnellement équivalent de celle-ci montrant une identité de séquence avec la CT-1 humaine d'au moins 90 % pour la préparation d'un médicament destiné au traitement d'une maladie métabolique, où la maladie métabolique est sélectionnée dans le groupe consistant en l'obésité, l'hyperglycémie, l'insulinorésistance, le diabète de type 2, et la dyslipidémie, où l'identité de séquence est déterminée en utilisant l'algorithme BLASTP.

3. Procédé cosmétique pour le traitement d'une surcharge pondérale de grade 1 (25,0-29,9 kg/m²), qui comprend l'administration à un patient d'une quantité pharmaceutiquement active d'un composé qui induit une activité de cardiotrophine-1 sélectionné dans le groupe consistant en
(i) la cardiotrophine-1 (CT-1),
(ii) un variant fonctionnellement équivalent de la CT-1 montrant une identité de séquence avec la CT-1 humaine d'au moins 90 %,
(iii) un polynucléotide qui code pour la CT-1 ou un variant fonctionnellement équivalent de celle-ci montrant une identité de séquence avec la CT-1 humaine d'au moins 90 %,
(iv) un vecteur qui comprend un polynucléotide selon (iii) et
(v) une cellule capable de sécréter dans le milieu la cardiotrophine-1 ou un variant fonctionnellement équivalent de celle-ci montrant une identité de séquence avec la CT-1 humaine d'au moins 90 % où l'identité de séquence est déterminée en utilisant l'algorithme BLASTP.

4. Composé pour son utilisation selon la revendication 1, utilisation selon la revendication 2 ou procédé selon la revendication 3, où ledit composé est administré pour un traitement aigu ou chronique.

5. Composition qui comprend, ensemble ou séparément, un composé qui induit une activité de cardiotrophine sélectionné dans le groupe consistant en
(i) la cardiotrophine-1 (CT-1),
(ii) un variant fonctionnellement équivalent de la CT-1 montrant une identité de séquence avec la CT-1 humaine d'au moins 90 %,
(iii) un polynucléotide qui code pour la CT-1 ou un variant fonctionnellement équivalent de celle-ci montrant une identité de séquence avec la CT-1 humaine d'au moins 90 %,
(iv) un vecteur qui comprend un polynucléotide selon (iii) et
(v) une cellule capable de sécréter dans le milieu la cardiotrophine-1 ou un variant fonctionnellement équivalent de celle-ci montrant une identité de séquence avec la CT-1 humaine d'au moins 90 % et un composé antidiabétique, où l'identité de séquence est déterminée en utilisant l'algorithme BLASTP.

6. Composition selon la revendication 5, dans laquelle le composé antidiabétique est sélectionné dans le groupe consistant en un composé qui induit une activité d'insuline et un composé qui induit l'activité hypoglycémique et/ou un agent sensibilisant à l'activité de l'insuline.

7. Composition selon la revendication 6, où le composé qui induit une activité d'insuline est sélectionné dans le groupe consistant en :
(i) l'insuline,
(ii) un polynucléotide qui code pour l'insuline et
(iii) un vecteur qui comprend un polynucléotide selon (ii).

8. Composition selon les revendications 5 ou 7 pour son utilisation dans le traitement d'une maladie métabolique, où la composition est administrée simultanément, séparément ou de manière séquentielle, et où la maladie métabolique est sélectionnée dans le groupe consistant en l'obésité, l'insulinorésistance, l'hyperglycémie, la dyslipidémie, et le diabète de type 2.

9. Utilisation d'une composition selon les revendications 5 ou 7 pour la préparation d'un médicament destiné au traitement d'une maladie métabolique, où la composition est administrée simultanément, séparément, ou de manière séquentielle.

10. Procédé cosmétique pour le traitement d'une surcharge pondérale de grade 1 (25,0-29,9 kg/m²), qui comprend l'administration au patient d'une quantité pharmaceutiquement active d'une composition selon les revendications 5 à 7.

11. Procédé selon la revendication 10, dans lequel le traitement est aigu ou chronique.
